# EUROPEAN PATENT APPLICATION

(11) **EP 4 541 357 A2**
(43) Date of publication of application: **23.04.2025**
(21) Application number: 23843367.6
(22) Date of filing: 19.07.2023
(51) Int. Cl.: A61K 31/765, A61K 33/04, A61K 31/375, A61K 33/14, A61K 9/00, A61P 1/10

(54) **BOWEL CLEANSING COMPOSITION WITH REDUCED DOSAGE ON THE DAY OF EXAMINATION**

(30) Priority: 20.07.2022 KR 20220089764; 18.07.2023 KR 20230092763
(71) Applicant: Kang, Yoon Sik, Seoul 06718 (KR); Shin, Chung Yoon, Seoul 06718 (KR); Kang, Hyun Suk, Gangnam-gu Seoul 06293 (KR); Kang, Hyun Yee, Seoul 06291 (KR)
(72) Inventor: Kang, Yoon Sik, Seoul 06718 (KR); Shin, Chung Yoon, Seoul 06718 (KR); Kang, Hyun Suk, Gangnam-gu Seoul 06293 (KR); Kang, Hyun Yee, Seoul 06291 (KR)
(74) Representative: BCKIP Part mbB
(86) International application number: PCT/KR2023/010399
(87) International publication number: WO 2024/019521

(57) **Abstract**

The present invention relates to a bowel cleansing composition and, specifically, to a bowel cleansing composition that reduces the dosage required on the day of examination requiring bowel cleansing, thereby increasing safety.

## Description

### [Technical Field]

The present disclosure relates to a bowel cleansing composition, and more particularly, to a bowel cleansing composition that dramatically improves safety and ease of dosage by reducing dosage on the day of an examination requiring bowel cleansing.

### [Background Art]

Colonoscopy is a highly effective means to completely prevent colon cancer. However, a screening rate for colonoscopy is low worldwide, and one of the biggest reasons for the low screening rate may be the difficulty of taking bowel cleansers. Unfortunately, it is recognized by many persons that most colon cleansers have too many doses, ranging from 2 to 4 L, and most of the flavors are disgusting, making them very difficult to take. In particular, all existing bowel cleansers are inconvenient in their dosing regimen in that a large amount of bowel cleansers or water, totaling 1 to 2 L, should be taken within a short period of time on the early morning on the day of the colonoscopy.

Furthermore, this dosing regimen of the existing bowel cleansers may also cause problems in the safety for the subject. First of all, two-thirds of colonoscopy patients also receive gastroscopy, and fasting for at least 8 hours, including water, is recommended during gastroscopy. Thus, there is a problem that it is directly contradicted by the method of taking bowel cleansers for colonoscopy. Furthermore, most endoscopies are performed under sedative anesthesia, and mixed solutions such as bowel cleansers require fasting for at least 3 hours before sedative anesthesia ("Easy sedation therapy for non-anesthesia pain medicine medical staff", Doo-jae Min, Department of Anesthesiology and Pain Medicine, Korea University Ansan Hospital, 2020).

In order to further activate colonoscopy and prevent the occurrence of colon cancer as much as possible through more accurate tests, there is a need for a new composition of bowel cleanser that reduces the total dose, improves flavor to make it more convenient to take, and increases safety by dramatically reducing the amount to take, especially on the early morning on the day of examination.

### [Disclosure]

### [Technical Problem]

An object of the present disclosure intends is to provide a bowel cleansing composition that exhibits excellent bowel cleansing effects while increasing safety by dramatically reducing the dosage on the day of the examination requiring bowel cleansing.

### [Technical Solution]

### <Summary of the invention>

The present disclosure provides a bowel cleansing composition taken prior to an examination requiring bowel cleansing, wherein
the composition is taken in divided doses the day before and on the day of the examination, and
a total dosage taken on the day of the examination is 700 mL or less.

The present disclosure also provides a method of cleansing a bowel, comprising administering to a human a bowel cleansing composition prior to an examination requiring bowel cleansing, wherein
the composition is taken in divided doses the day before and on the day of the examination, and
total dose (or dosage) taken on the day of the examination is 700 mL or less.

The present disclosure also provides a bowel cleansing composition for use prior in taking prior to an examination requiring bowel cleansing, wherein
the composition is taken in divided doses the day before and on the day of the examination, and
a total dosage taken on the day of the examination is 700 mL or less.

In an embodiment, the total dosage or dose taken or administered on the day of the examination may be 600 mL or less.

In an embodiment, the total dosage or dose taken or administered on the day of the examination may be 500 mL or less.

In an embodiment, the total dosage or dose taken or administered on the day of the examination may be 360, 400, 450, 480, 500, 568 or 600 mL.

In some embodiments, the bowel cleansing composition may have the total dosage or dose of 1.0 L to 4.0 L taken or administered on the day of the examination.

In an embodiment, the bowel cleansing composition comprises a preparation solution obtained by dissolving a composition in a form of a powder, granule, liquid preparation, tablet, or capsule containing the effective ingredient is a water-soluble solvent; and separately a water-soluble solvent, and
the bowel cleansing composition is administered in a mode by taking or administering the preparation solution, followed by taking or administering the water-soluble solvent separately, wherein the mode may be repeated a total of two to four times on the day before and on the day of the examination.

In an embodiment, the bowel cleansing composition may be in the form of any one of a solution, a suspension, and an emulsion.

In an embodiment, the bowel cleansing composition comprises
one or more sugar alcohols selected from xylitol, sorbitol, glycerol, erythritol, threitol, arabitol, ribitol, mannitol, galactitol, fucitol, iditol, inositol, volemitol, isomalt, maltitol, lactitol, maltotriitol, maltotegratol, and polyglycitol as a first effective ingredient;
ascorbic acid or a mixture of ascorbic acid and ascorbate salts as a second effective ingredient; and
a water-soluble solvent,
wherein the content of the first effective ingredient may be from 10 g/L to 500 g/L based on the total composition, the content of the second effective ingredient may be from 1 g/L to 300 g/L based on the total composition, and the content of the water-soluble solvent may be from 1.0 to 3.0 L.

In a specific embodiment, the bowel cleansing composition further comprises
sodium bicarbonate and/or potassium bicarbonate as third effective ingredient,
wherein the content of the third effective ingredient may be from 0.5 g/L to 5 g/L based on the total composition.

In a more specific embodiment, the bowel cleansing composition comprises
110 g of D-sorbitol;
15 g of ascorbic acid;
2.1 g of sodium bicarbonate;
0.9 g of potassium bicarbonate; and
1.0 L to 3.0 L of water,
wherein the water may be administered in the form of a solution in which the ingredients are dissolved, or may be administered alone.

In a more specific embodiment, the bowel cleansing composition comprises
1.0 L to 1.5 L of water, or a total dosage taken on the day before and on the day of the examination is 1.0 L to 1.5 L, and
the total dosage taken on the day of the examination may be 500 mL or less.

In a more specific embodiment, the bowel cleansing composition comprises
1.28 L, 1.35 L, or 1.44 L of water, or a total dosage taken the day before and on the day of the examination may be 1.28 L, 1.35 L, or 1.44 L, and
the total dosage taken on the day of the examination may be 360 mL, 460 mL or 480 mL.

In an embodiment, the bowel cleansing composition comprises
polyethyleneglycol as a first effective ingredient;
ascorbic acid or a mixture of ascorbic acid and ascorbate salts as a second effective ingredient; and
a water-soluble solvent,
wherein the content of the first effective ingredient may be from 10 g/L to 500 g/L based on the total composition, the content of the second effective ingredient may be from 1 g/L to 300 g/L based on the total composition, and the content of the water-soluble solvent may be from 1.0 to 3.0 L.

In a specific embodiment, the bowel cleansing composition further comprises
one or more selected from the group consisting of sodium chloride, potassium chloride, and sodium sulfate as a third effective ingredient,
wherein the content of the third effective ingredient may be from 0.5 g/L to 50 g/L based on the total composition.

In a more specific embodiment, in the bowel cleansing composition,
the polyethylene glycol may have an average molecular weight of 3000 to 4000 Da.

In a more specific embodiment, the bowel cleansing composition comprises
200 g of polyethylene glycol 3350;
9.4 g of ascorbic acid;
11.8 g of sodium ascorbate;
15 g of anhydrous sodium sulfate;
5.382 g of sodium chloride;
2.030 g of potassium chloride; and
1.0 to 3.0 L of water,
wherein the water may be administered in the form of a solution in which the ingredients are dissolved, or may be administered alone.

In a more specific embodiment, the bowel cleansing composition comprises
3.0 L of water, or a total dosage taken the day before and on the day of the examination may be 3.0 L, and
the total dosage taken on the day of the examination may be 600 mL.

In a more specific embodiment, the bowel cleansing composition comprises
140 g of polyethylene glycol 3350;
7.54 g of ascorbic acid;
48.11 g of sodium ascorbate;
9 g of anhydrous sodium sulfate;
5.2 g of sodium chloride;
2.2 g of potassium chloride; and
water 1.0 to 3.0 L,
wherein the water may be administered in the form of a solution in which the ingredients are dissolved, or may be administered alone.

In a more specific embodiment, the bowel cleansing composition comprises
2.0 L of water, or a total dosage taken the day before and on the day of the examination may be 2.0 L, and
the total dosage taken on the day of the examination may be 460 mL or 500 mL.

In a more specific embodiment, the bowel cleansing composition comprises
160 g of polyethylene glycol 3350;
40.6 g of ascorbic acid;
9.4 g of sodium ascorbate;
18 g of anhydrous sodium sulfate;
2.7 g of sodium chloride;
1 g of potassium chloride; and
1.0 to 3.0 L of water,
wherein the water may be administered in the form of a solution in which the ingredients are dissolved, or may be administered alone.

In a more specific embodiment, the bowel cleansing composition comprises
2.0 L of water, or a total dosage taken the day before and on the day of the examination may be 2.0 L, and
the total dosage taken on the day of the examination may be 400 mL.

In an embodiment, the bowel cleansing composition may comprise
based on the total composition,
0.001 g/L to 0.1 g/L of sodium picosulfate or a hydrate thereof;
0.5 g/L to 50 g/L of magnesium oxide;
1 g/L to 50 g/L of citric acid; and
1.0 to 3.0 L of a water-soluble solvent.

In a specific embodiment, the bowel cleansing composition comprises
0.03 g of sodium picosulfate hydrate;
10.5 g of magnesium oxide;
36.0 g of citric acid; and
1.0 to 3.0 L of water,
wherein the water may be administered in the form of a solution in which the ingredients are dissolved, or may be administered alone.

In a more specific embodiment, the bowel cleansing composition comprises
3.0 L of water, or a total dosage taken the day before and on the day of the examination may be 3.0 L, and
the total dosage taken on the day of the examination may be 600 mL.

In an embodiment, the bowel cleansing composition may comprise, based on the total composition,
10 g/L to 500 g/L of polyethylene glycol;
1 g/L to 300 g/L of sorbitol;
0.001 g/L to 1 g/L of sodium picosulfate or a hydrate thereof; and
1.0 to 3.0 L of a water-soluble solvent.

In a specific embodiment, the bowel cleansing composition comprises
75 g/L of polyethylene glycol;
27.3 g/L of sorbitol;
0.005 g/L of sodium picosulfate or a hydrate thereof; and
1.0 to 3.0 L of water,
wherein the water may be administered in the form of a solution in which the ingredients are dissolved, or may be administered alone.

In a more specific embodiment, the bowel cleansing composition comprises
2.0 L of water, or a total dosage taken the day before and on the day of the examination may be 2.0 L, and
the total dosage taken on the day of the examination may be 400 mL.

In an embodiment, the bowel cleansing composition may comprise, based on the total composition,
5 g/L to 300 g/L of sodium sulfate;
0.1 g/L to 50 g/L of potassium sulfate;
0.1 g/L to 50 g/L of magnesium sulfate; and
1.0 to 3.0 L of a water-soluble solvent.

In a specific embodiment, the bowel cleansing composition comprises
12.333 g/L of anhydrous sodium sulfate;
2.206 g/L of potassium sulfate;
1.128 g/L of magnesium sulfate; and
1.0 to 3.0 L of water, and
wherein the water may be administered in the form of a solution in which the ingredients are dissolved, or may be administered alone.

In a more specific embodiment, the bowel cleansing composition comprises
2.838 L of water, or a total dosage taken the day before and on the day of the examination may be 2.838 L, and
the total dosage taken on the day of the examination may be 568 mL.

Throughout the present specification, when a part "comprises" a certain component, this does not mean excluding other components, unless specifically stated to the contrary, and means further comprising other components.

As used herein, the term "about" means within 10% of a given value or range, preferably within 5%, and more preferably within 1%.

The present disclosure relates to bowel cleansing composition which dramatically reduces the amount of drinking on the day of an examination requiring bowel cleansing, for example, in the early morning, thereby increasing safety, and more particularly, to bowel cleansing composition wherein the total dosage of the bowel cleansing composition (including any co-solvent such as water, etc.) taken on the day of the examination is about 700 mL or less, such as about 200 to about 700 mL, preferably about 600 mL or less, and more preferably about 500 mL.

In an embodiment, the present disclosure provides a bowel cleansing composition taken prior to an examination requiring bowel cleansing, and provides bowel cleansing composition, wherein
the composition is taken in divided doses the day before and on the day of the examination, and
a total dose taken on the day of the examination is 700 mL or less.

In another embodiment, the present disclosure provides to a method of cleansing a bowel, comprising administering to a human a bowel cleansing composition prior to an examination requiring bowel cleansing,
wherein the composition is taken in divided doses the day before and on the day of the examination, and
a total dosage/dose taken on the day of the examination is 700 mL or less.

The total dosage or dose taken on the day of the examination is preferably 600 mL, and more preferably 500 mL. For example, the dosage may be about 300, about 310, about 320, about 330, about 340, about 350, about 360, about 370, about 380, about 390, about 400, about 410, about 420, about 430, about 440, about 450, about 460, about 470, about 480, about 490, about 500, about 510, about 520, about 530, about 540, about 550, about 560, about 570, about 580, about 590, about 600, about 610, about 620, about 630, about 640, about 650, about 660, about 670, about 680, about 690, about 700 mL. In an embodiment, the total dosage taken on the day of the examination may be about 360, about 400, about 450, about 480, about 500, about 568, or about 600 mL. In this case considering the numerical range of the term "about" as defined herein, the above values also include dosages within 10%, preferably within 5%, and even more preferably within 1% of the above specific values.

As used herein, the term "dosage" or "dose" may be used interchangeably if the meaning does not change in particular in interpretation.

The phrase "examination requiring/needing bowel cleansing" herein refers to a colonoscopy, X-ray examination, such as enterography, radiography, or an examination that requires emptying the inside of the bowel as completely as possible in preoperative bowel cleansing etc. (e.g., treatment before colon surgery, treatment before anal surgery such as hemorrhoids), and includes, without limitation, any examination or procedure that requires bowel cleansing in addition to those listed above. In addition to the above, other treatments may include the treatment of diseases such as chronic or acute constipation, etc., if needed. In particular, the examination also includes cases where a gastroscopy is performed at the same time as a colonoscopy.

As used herein, the phrase "bowel cleansing composition" refers to a "purgative" or a substance that promotes defecation, and includes the category of diarrheal action. For example, the phase "bowel cleansing" includes not only a defecation that completely empties the colon ("complete purgation") or a defection that almost completely empties the colon (stronger catharsis), but also a defecation causing a mild catharsis that results in diarrhea ("partial purgation"). As used herein, the phrase "bowel cleansing composition" comprehensively includes a composition used to cleanse the bowel or a composition used to manufacture a bowel cleansing medicine. Similarly, as used herein, the phrase "method of cleansing the bowel" refers to a method of cleansing the bowel using the "bowel cleansing composition".

In an embodiment, the composition according to the present disclosure may be administered in the form of a preparation solution with a co-solvent, such as water, etc., or may be administered as is.

In another embodiment, the bowel cleansing composition according to the present disclosure may be understood as comprising a first part comprising an effective ingredient; and a second part comprising a co-solvent, such as a water-soluble solvent, such as water. In this case, it is understood that the first part comprises an ingredient having bowel cleansing effect, and any pharmaceutically acceptable excipient, etc., in addition to the effective ingredient directly having a bowel cleansing effect. The first part may be selected from any pharmaceutically acceptable form, for example, powder, granular, liquid formulation, tablet, capsule, etc., and may preferably be in a form that may be dissolved in the second part, for example, powder, granular, or liquid formulation. If the first part is in the form of a tablet or capsule, etc., it may be taken by dissolving it in water or an auxiliary solution in the form of a preparation solution, or may be taken separately as a tablet or capsule with water or an auxiliary solution, etc. In this case, the dosage of a solution of 700 mL or less according to the present disclosure will be determined by the amount of water or an auxiliary solution taken together with the tablet or capsule, etc.

The second part may be used, without limitation, as long as it is a co-solvent capable of dissolving the first part, preferably, a water-soluble solvent, for example, water, carbonated water, alkaline ionized water, beverages, and in particular water may be used. Additionally, there is no particular limitation on the type of beverage as long as it may achieve the effect of the present disclosure, for example, drip coffee, various juices, cola, cider, gin, and tonic, etc. may be used as needed.

The second part may be administered as a liquid formulation by dissolving the first part, or the second part may be administered alone. A liquid formulation in which the first part is dissolved in the second part, i.e., a solution in which the effective ingredient and/or other excipients are well mixed with each other by content, and then mixed with an appropriate amount of co-solvent such as water, is sometimes referred to as a "preparation solution".

In an embodiment, the bowel cleansing composition according to the present disclosure may comprise a liquid formulation (preparation solution) obtained by dissolving a composition (first part) in the form of a powder, granule, or liquid formulation containing an effective ingredient and a pharmaceutically acceptable excipient in a co-solvent, e.g., a water-soluble solvent; and separately a co-solvent, e.g., a water-soluble solvent. In this case, the bowel cleansing composition may be administered by taking a preparation solution in the form of the liquid formulation and then separately taking the water-soluble solvent.

Accordingly, the bowel cleansing composition according to the present disclosure, in particular, the "preparation solution" or the liquid formulation may be in the form of a solution, suspension, or emulsion. However, the bowel cleansing composition according to the present disclosure is administered by taking the water-soluble solvent separately from the preparation solution, and in some cases, both the preparation solution and the water-soluble solvent, each taken separately, may be simply referred to collectively as a solution, suspension, or emulsion.

The bowel cleansing composition according to the present disclosure may be taken in a total dosage all at one time (non-divided administration) or divided into multiple time points (divided administration). Specifically, a non-divided administration is one that is administered over the course of the day (i.e., divided), for example, may be taken the evening before the examination or early morning on the day of the examination, over several hours. Specifically, one may take a portion of the total dosage and then take the remainder after a certain period of time, for example, 0.5 to 3 hours later. Divided administration means administering the dosage in two separate days, the day before and on the day of the examination, for example, administering part of the dosage on the evening before the examination and the rest of the dosage on the early morning the day of the examination.

In an embodiment, the composition according the present disclosure may be administered in two or more doses the day before and/or the day of the examination, preferably in two divided doses, the night before the examination; and the early morning on the day of the examination. The number of doses "two or more times" as used herein is based on the dose of the preparation solution and for example, if water is taken separately after taking a certain amount of preparation solution the night before the examination, this is considered one dose. In another example, if one takes a certain amount of the preparation solution and then water separately the night before the examination (first dose), and repeat the process after a certain amount of time (e.g., 30 minutes, 1 hour, or 1.5 hours), and then take a certain amount of the preparation solution and water separately (second does), each of these is treated as a separate dose, and this is considered as a total 2 doses. However, taking a certain amount of the preparation solution determined as a single dose in several divided doses (for example, taking it with breaks at 5-10 minutes intervals because it is difficult for the user to take it all at once) is considered to be a single dose.

In an embodiment, the composition according to the present disclosure is administered in a mode by taking the preparation solution (liquid formulation) and then separately taking the water-soluble solvent, which may be repeated a total of two to four times on the day before and on the day of the examination. However, "taking the water-soluble solvent separately" includes not only taking the water-soluble solvent after taking the liquid formulation completely, but also taking the water-soluble solvent in between taking the liquid formulation.

The amount of the preparation solution or co-solvent, such as water, may be appropriately selected by those skilled in the art, provided that the total solution dosage on the day of the examination (e.g., several hours prior to the examination, e.g., prior to 3 hours, prior to 2 hours, or prior to 1 hour of the examination), including all the preparation solution and the co-solvent, such as water, is about 700 mL or less, e.g., from about 200 to about 700 mL, preferably about 600 mL or less, and even more preferably about 500 mL or less.

Meanwhile, the total dosage of the bowel cleansing composition according to the present disclosure may be any value between about 1.0 and about 5.0 L, such as between about 1.0 and about 4.0 L, and specifically between about 1.0 and about 3.0 L. In an embodiment, the total dosage may be about 1.0 L, about 1.28 L, about 1.35 L, about 1.44 L, about 2.0 L, about 2.838 L, about 3.0 L, etc. In this case, the total dosage may refer to the dosage taken the day before and on the day of the examination. Accordingly, the total dosage of the bowel cleansing composition according to the present disclosure taken the day before and on the day of the examination may be the above value, for example, about 1.0 L to about 4.0 L.

In an embodiment, the bowel cleansing composition according to the present disclosure may comprise as an effective ingredient one or more of the following ingredients.
- Polyethylene glycol (PEG), for example, PEG having a number average molecular weight of 1,000 to 10,000, preferably PEG having a number average molecular weight of 2,000 to 6,000, more preferably PEG having a number average molecular weight of 3,000 to 5,000, specifically, polyethylene glycol 3350;
- Ascorbic acid or a salt thereof, for example, an alkaline metal (e.g., sodium or potassium) or an alkaline earth metal salt (e.g., calcium) of ascorbic acid, with a specific example being sodium ascorbate;
- one or more sugar alcohols selected from xylitol, sorbitol (e.g., D-sorbitol), glycerol, erythritol, threitol, arabitol, ribitol, mannitol, galactitol, fucitol, iditol, inositol, volemitol, isomalt, maltitol, lactitol, maltotriitol, maltotegratol, and polyglycitol;
- picosulfate, such as sodium picosulfate or a hydrate thereof;
- citric acid;
- magnesium oxide;
- (anhydrous) sodium sulfate;
- potassium sulfate;
- (anhydrous) magnesium sulfate;
- sodium chloride;
- anhydrous sodium hydrogen phosphate;
- sodium dihydrogen phosphate monohydrate; and
- potassium chloride.

The bowel cleansing composition according to the present disclosure may further comprise any pharmaceutically acceptable excipients or additives suitable for bowel cleansers. The excipients or additives are not particularly limited as long as they are those customarily used in bowel cleansing compositions.

For example, if bowel cleansing composition comprises ascorbic acid as an effective ingredient, it may be difficult to take due to its high acidity and excessive sourness, so it may additionally comprise a bicarbonate, such as sodium bicarbonate or potassium bicarbonate, as an ingredient to neutralize it.

In addition, the bowel cleansing composition according to the present disclosure may comprise an appropriate amount of antioxidant to prevent oxidation of the ascorbic acid. Such antioxidants may include, for example, but are not limited to, amino acids such as ferulic acid, glycine and histidine, hyaluronic acid, tocopherols, etc.

The bowel cleansing composition according to the present disclosure may also comprise sweeteners to improve flavor, and available sweeteners may include, for example, but are not limited to, sucralose, maltodextrin, glucose, sucrose, dextrose, saccharin, aspartame, stevia, etc.

In addition, the bowel cleansing composition according to the present disclosure may comprise an appropriate amount of edible fruit flavor to increase the compliance. These fruit flavors may include strawberry, orange, apple, grape, lemon, banana, and cherry flavors.

Further, the bowel cleansing composition according to the present disclosure may further comprise an adsorbent for adsorbing trace amounts of iron ions and copper ions likely to be contained in the water-soluble solvent. Examples of such adsorbents may include, but are not limited to, Versene^{™} CA chelating agent (Dow Chemical Company).

Also, the bowel cleansing composition according to the present disclosure may further comprise a bubble formulation inhibitor to eliminate bowel gas or to eliminate bowel bubbles that often occur after bowel cleansing. In one aspect of the present disclosure, simethicone may be used as a bubble formulation inhibitor, but the present disclosure is not limited thereto. Simethicone used as a bubble formulation inhibitor in the present disclosure, may be mixed into the bowel cleansing composition in the form of a suspension. Its usage may be in accordance with methods known in the art.

The bowel cleansing composition according to the present disclosure may further comprise the known cleansing components or auxiliary components to enhance the cleansing effect. Additional cleansing ingredients and cleansing-enhancing auxiliary ingredients may include, for example, but are not limited to, citric acid, magnesium such as magnesium oxide, sodium docusate, senna extract (such as sennoside), aloe extract (such as aloin), pectin, zinc such as zinc oxide, caffeine, etc.

The bowel cleansing composition according to the present disclosure may be PEG-based, PSMC-based, oral sulfate-based, or Sorbitol-based.

**[Table 1]**

| | |
|---|---|
| PEG-based | A bowel cleanser containing all or part of PEG 3350, sodium ascorbate, ascorbic acid, anhydrous sodium sulfate, potassium chloride, and sodium chloride as effective ingredients |
| PSMC-based | Bowel cleanser containing part or all of citric acid, magnesium oxide, and sodium picosulfate hydrate as effective ingredients |
| Oral sulfate-based | A bowel cleanser containing part or all of anhydrous magnesium sulfate, potassium sulfate, and anhydrous sodium sulfate as effective ingredients |
| Sorbitol/Mannitol-based | Bowel cleanser containing part or all of sorbitol/mannitol, ascorbic acid, sodium picosulfate, and PEG as effective ingredients |

Specifically, the bowel cleansing composition according to the present disclosure may comprise the following effective ingredients. In this case, the following compositions may be taken in two or more divided doses the day before or on the day of the examination, and the amount of the preparation solution or co-solvent such as water may be appropriately selected by those skilled in the art, provided that the total dosage of the solution taken on the day of the examination (e.g., several hours before the examination), including all the preparation solution and the co-solvent such as water, is about 700 mL or less, such as from about 200 to about 700 mL, preferably about 600 mL or less, and more preferably about 500 mL or less.

### [Composition example I]

1) A composition comprising polyethylene glycol 3350; ascorbic acid, or sodium ascorbate; and anhydrous sodium sulfate (which may additionally comprise potassium chloride, sodium chloride, or other additives) as effective ingredients.
2) A composition comprising polyethylene glycol 3350 and sodium chloride as effective ingredients.
3) A composition comprising D-sorbitol/mannitol; and ascorbic acid or sodium ascorbate as effective ingredients.
4) A composition comprising anhydrous sodium dihydrogen phosphate; and sodium dihydrogen phosphate monohydrate as effective ingredients.
5) A composition comprising sodium sodium picosulfate hydrate; magnesium oxide; and citric acid as effective ingredients.
6) A composition comprising anhydrous sodium sulfate; and anhydrous magnesium sulfate (which may further comprise potassium sulfate) as effective ingredients.
7) A composition comprising D-sorbitol; polyethylene glycol 3350; and sodium picosulfate hydrate as effective ingredients.
8) A composition comprising D-sorbitol/mannitol; ascorbic acid or sodium ascorbate; sodium bicarbonate; and potassium bicarbonate as effective ingredients.

In one specific embodiment, the bowel cleansing composition according to the present disclosure may comprise the following effective ingredients.

### [Composition example II]

1) A composition comprising 100 to 400 g of polyethylene glycol 3350; 10 to 80 g of ascorbic acid or sodium ascorbate; and 5 to 40 g of anhydrous sodium sulfate (which may additionally comprise 1 to 5 g of potassium chloride, 1 to 10 g of sodium chloride, and other additives) . The composition is taken in divided portions of a total of about 1 L to 2 L of the preparation solution and a total of about 1 L of water, and the total dosage of the solution taken on the day of the examination, including all the preparation solution and water, is about 700 mL or less, preferably about 600 mL or less, and more preferably about 500 mL or less.
2) A composition comprising 350 to 450 g of polyethylene glycol 3350 and 10 to 15 g of sodium chloride as effective ingredients. The composition is taken in divided portions of a total of about 4 L of the preparation solution, and the total dosage of the solution taken on the day of the examination, including all the preparation solution and water, is about 700 mL or less, preferably about 600 mL or less, and more preferably about 500 mL or less.
3) A composition comprising 50 to 150 g of D-sorbitol, 5 to 30 g of ascorbic acid or sodium ascorbate as an effective ingredient. The composition is taken in divided portions of about 0.5 L of the preparation solution and about 1.0 L of water, and the total dosage of the solution taken on the day of the examination, including all the preparation solution and water, is about 700 mL or less, preferably about 600 mL or less, and more preferably about 500 mL or less.
4) A composition comprising 10 to 20 g of anhydrous sodium dihydrogen phosphate and 30 to 50 g of sodium dihydrogen phosphate monohydrate as effective ingredients. If the composition is in the form a tablet, it is taken in divided doses with about 2 L of water, and the total dosage of water taken on the day of the examination, is about 700 mL or less, preferably about 600 mL or less, and more preferably about 500 mL or less.
5) A composition comprising 20 to 50 mg of sodium picosulfate hydrate, 5 to 15 g of magnesium oxide, and 30 to 50 g of citric acid as effective ingredients. The composition is taken in divided portions of about 0.3 L to 0.4 L of the preparation solution and total 2 L of water, and the total dosage of the solution on the day of the examination, including all the preparation solution and water, is about 700 mL or less, preferably about 600 mL or less, and more preferably about 500 mL or less.
6) A composition comprising 25 to 45 g of anhydrous sodium sulfate, 2 to 5 g of anhydrous magnesium sulfate (which may additionally comprise 1 to 10 g of potassium sulfate) as effective ingredients. If the composition is a tablet, it is taken in divided portions with about 2.5 to 3.0 L of water and if the composition is a powder, it is taken in divided portions with about 0.9 to 1.0 L of the preparation solution and about 1.8 to 2.0 L of water, and the total dosage of the solution taken on the day of the examination, including all the preparation solution and water, is about 700 mL or less, preferably about 600 mL or less, and more preferably about 500 mL or less.
7) A composition comprising 40 to 100 g of D-sorbitol, 100 to 300 g of polyethylene glycol 3350, and 5 to 20 mg of sodium picosulfate hydrate as effective ingredients. The composition is taken in divided portions with about 1 L of the preparation solution and about 1.0 L of water, and the total dosage of the solution on the day of the examination, including all the preparation solution and water, is about 700 mL or less, preferably about 600 mL or less, and more preferably about 500 mL or less.
8) A composition comprising 50 to 150 g of D-sorbitol; 10 to 20 g of ascorbic acid or sodium ascorbate; 1 to 5 g of sodium bicarbonate; and 0.5 to 1.5 g of potassium bicarbonate as effective ingredients. The composition is taken in divided portions with about 0.8 to 1.1 L of the preparation solution and about 0.3 to 0.5 L of water, and the total dosage of the solution taken on the day of the examination, including all the preparation solution and water, is about 700 mL or less, preferably about 600 mL or less, and more preferably about 500 mL or less.

In a more specific embodiment, the bowel cleansing composition according to the present disclosure may comprise the following effective ingredients. In this case, the following compositions may be taken in two or more divided doses the day before or on the day of the examination, and the amount of the preparation solution or co-solvent such as water may be appropriately selected by those skilled in the art, provided that the total dosage of the solution taken on the day of the examination, including all the preparation solution and the co-solvent such as water, is 700 mL or less, such as from 200 to 700 mL, preferably about 600 mL or less, and more preferably about 500 mL or less.

**[Table 2]**

| [Composition example III] | |
|---|---|
| **No.** | **Composition (effective ingredient)** |
| 1 | A composition comprising 100 to 250 g of polyethylene glycol 3350; 20 to 25 g of ascorbic acid or sodium ascorbate, 10 to 20 g of anhydrous sodium sulfate (which may additionally comprise 1 to 5 g of potassium chloride, 1 to 10 g of sodium chloride, and other additives) |
| 2 | A composition comprising 100 to 200 g of polyethylene glycol 3350; 40 to 70 g of ascorbic acid or sodium ascorbate; and 5 to 15 g of anhydrous sodium sulfate (which may additionally comprise 1 to 5 g of potassium chloride, 1 to 10 g of sodium chloride, and other additives) |
| 3 | A composition comprising 100 to 200 g of polyethylene glycol 3350, 40 to 70 g of ascorbic acid or sodium ascorbate; and 10 to 20 g of anhydrous sodium sulfate (which may additionally comprise 1 to 5 g of potassium chloride, 1 to 10 g of sodium chloride, and other additives) |
| 4 | A composition comprising 150 to 250 g of polyethylene glycol 3350; 10 to 30 g of ascorbic acid or sodium ascorbate, 10 to 30 g of anhydrous sodium sulfate (which may additionally comprise 1 to 5 g of potassium chloride, 1 to 10 g of sodium chloride, and other additives) |
| 5 | A composition comprising 200 to 300 g of polyethylene glycol 3350 and 20 to 30 g of anhydrous sodium sulfate |
| 6 | A composition comprising 350 to 450 g of polyethylene glycol 3350 and 10 to 15 g of sodium chloride |
| 7 | A composition comprising 50 to 150 g of D-sorbitol and 10 to 30 g of ascorbic acid or sodium ascorbate |
| 8 | A composition comprising 40 to 100 g of D-sorbitol, 150 g of polyethylene glycol 3350, and 5 to 20 mg of sodium picosulfate hydrate |
| 9 | A composition comprising 10 to 20 g of anhydrous sodium dihydrogen phosphate and 30 to 50 g of sodium dihydrogen phosphate monohydrate |
| 10 | A composition comprising 20 to 50 mg of sodium picosulfate hydrate, 5 to 15 g of magnesium oxide, and 30 to 50 g of citric acid |
| 11 | A composition comprising 25 to 45 g of anhydrous sodium sulfate and 2 to 5 g of anhydrous magnesium sulfate |
| 12 | A composition comprising 25 to 45 g of anhydrous sodium sulfate, 2 to 5 g of anhydrous magnesium sulfate, and 5 to 10 g of potassium sulfate |
| 13 | A composition comprising 10 to 150 g of D-sorbitol; 10 to 20 g of ascorbic acid or sodium ascorbate; 1 to 3 g of sodium bicarbonate; and 0.5 to 1.0 g of potassium bicarbonate as effective ingredients |

In a more specific embodiment, the bowel cleansing composition according to the present disclosure may comprise the following effective ingredients. The following compositions may be taken in two or more divided doses the day before or on the day of the examination, and the amount of the preparation solution or co-solvent such as water may be appropriately selected by those skilled in the art, provided that the total dosage of the solution taken on the day of the examination, including all the preparation solution and the co-solvent such as water, is 700 mL or less, such as from 200 to 700 mL, preferably about 600 mL or less, and more preferably about 500 mL or less.

**[Table 3]**

| [Composition example IV] | | |
|---|---|---|
| **No.** | **Components** | **Content (g)** |
| 1 | Polyethylene glycol 3350 | 200.000 |
| | Anhydrous sodium sulfate | 15.000 |
| | Ascorbic acid | 9.400 |
| | Sodium ascorbate | 11.800 |
| | Sodium chloride | 5.382 |
| | Potassium chloride | 2.030 |
| 2 | Polyethylene glycol 3350 | 140 |
| | Anhydrous sodium sulfate | 9 |
| | Potassium chloride | 2.2 |
| | Sodium chloride | 5.2 |
| | Sodium ascorbate | 48.11 |
| | Ascorbic acid | 7.54 |
| 3 | D-sorbitol | 100 |
| | Ascorbic acid | 15 |
| | Sodium bicarbonate | 2.1 |
| | Potassium bicarbonate | 0.9 |
| 4 | Sodium picosulfate hydrate | 0.03 |
| | Hard magnesium oxide | 10.5 |
| | Citric acid | 36 |
| 5 | Anhydrous sodium sulfate | 35 |
| | Potassium sulfate | 6.26 |
| | Anhydrous magnesium sulfate | 3.2 |
| 6 | D-sorbitol | 75.80 |
| | Ascorbic acid | 10 |
| | Sodium picosulfate hydrate | 0.02 |
| 7 | Ascorbic acid | 40.6 |
| | Sodium ascorbate | 9.4 |
| | Polyethylene glycol 3350 | 160 |
| | Potassium chloride | 1 |
| | sodium chloride | 2.7 |
| | Anhydrous sodium sulfate | 18 |
| 8 | Anhydrous sodium hydrogen phosphate | 12.736 |
| | sodium dihydrogen phosphate monohydrate | 35.264 |
| 9 (1) | citrate hydrate | 24 |
| | Magnesium oxide | 7.0 |
| | Sodium picosulfate hydrate | 0.02 |
| 9 (2) | Citrate hydrate | 36 |
| | Magnesium oxide | 10.5 |
| | Sodium picosulfate hydrate | 0.03 |
| 10 | Anhydrous magnesium sulfate | 2.88008 |
| | Potassium sulfate | 5.62996 |
| | Simethicon | 0.32004 |
| | Anhydrous sodium sulfate | 31.5 |
| 11 | D-sorbitol | 54.6 |
| | Polyethylene glycol 3350 | 150 |
| | Sodium picosulfate hydrate | 0.01 |
| 12 (powder) | Anhydrous magnesium sulfate | 3.2 |
| | Potassium sulfate | 6.26 |
| | Anhydrous sodium sulfate | 35 |
| 13 (Liquid) | Anhydrous magnesium sulfate | 3.2 |
| | Potassium sulfate | 6.26 |
| | Anhydrous sodium sulfate | 35 |
| 14 | Ascorbic acid | 9.4 |
| | Sodium ascorbate | 11.8 |
| | Polyethylene glycol 3350 | 200 |
| | Potassium chloride | 2.03 |
| | Sodium chloride | 5.382 |
| | Anhydrous sodium sulfate | 15 |
| 15 | Polyethylene glycol 3350 | 236 |
| | Potassium chloride | 2.96 |
| | Sodium bicarbonate | 6.72 |
| | Sodium chloride | 5.84 |
| | Anhydrous sodium sulfate | 22.8 |
| 16 | Polyethylene glycol 3350 | 420 |
| | Potassium chloride | 1.48 |
| | Sodium bicarbonate | 5.72 |
| | Sodium chloride | 11.2 |
| 17 | D-sorbitol | 110.000 |
| | Ascorbic acid | 15.000 |
| | Sodium bicarbonate | 2.100 |
| | Potassium bicarbonate | 0.900 |

In an embodiment, the composition according to the present disclosure may be packaged in separate packaging units in a number of portions depending on the convenience of administration, or may be provided in the form of kits packaged separately for each ingredient. In particular, ascorbic acid may be packaged separately from other ingredients depending on its chemical nature. In this case, the kit may include instructions for use that describes the dosing regimen or method of use mentioned throughout the specification of the present disclosure. In another embodiment, the bowel cleansing composition according to the present disclosure may be provided in the form of an appropriately packaged kit, with the total dosage divided according to the number of doses. Specifically, the bowel cleansing composition according to the present disclosure may be provided in a single package for a total dosage, and may be provided in two or more divided packages for a total dosage.

In an embodiment, the bowel cleansing composition according to the present disclosure may be packaged in one-piece (i.e., liquid) form with the first and second components and, if necessary, all components of third cleansing components, bubble formulation inhibitors, gastrointestinal stabilizers, and other additives with water-soluble solvents.

In another embodiment, the bowel cleansing composition according to the present disclosure may be provided in the form of a kit packaged in multiple packaging units. For example, the first component and the second component may be packaged together and the water-soluble solvent may be packaged separately, or the second component and the water-soluble solvent may be packaged together and the first cleaning component may be packaged separately, or the first component and the water-soluble solvent may be packaged together and the second cleaning component may be packaged separately, or the first cleaning ingredient and a portion of the water-soluble solvent may be packaged together and the second ingredient and a portion of the water-soluble solvent may be packaged together, or the first and second ingredients may be packaged together with a portion of the water-soluble solvent and the remainder of the water-soluble solvent may be packaged separately, and the water-soluble solvent may be any commercially available product (e.g., bottled water, sparkling water, etc.) used as it, but is not limited thereto. Additionally, third cleaning ingredients, bubble formulation inhibitors, gastrointestinal stabilizers, and other additives may be blended in various two-component forms, if necessary.

For example, some examples of the compositions according to the present disclosure may be provided in the form of a kit packaged as follows.

**[Table 4]**

| | |
|---|---|
| 1 | 50 g of polyethylene glycol 3350, 0.5075 g of potassium chloride, 1.3455 g of sodium chloride, 3.75 g of anhydrous sodium sulfate, 2.35 g of ascorbic acid, and 2.95 g of sodium ascorbate → 4 packets each |
| 2 | Dose 1: 100 g of polyethylene glycol 3350, 1 g of potassium chloride, 2 g of sodium chloride, 9 g of anhydrous sodium sulfate |
| | Dose 2: 7.54 g of ascorbic acid, 44.11 of sodium ascorbate, 1.2 g of potassium chloride, 40 g of polyethylene glycol 3350, and 3.2 g of sodium chloride |
| 3 | 10.15 g of ascorbic acid, 2.35 g of sodium ascorbate, 40 g of polyethylene glycol 3350, 0.25 g of potassium chloride, 0.675 g of sodium chloride, and 4.5 g of anhydrous sodium sulfate → 4 packets each |
| 4 | 398 mg of anhydrous disodium hydrogen phosphate and 1102 mg of sodium dihydrogen phosphate monohydrate → 32 tablets each |
| 5 | 12 g of citric acid, 3.5 g of hard magnesium oxide, and 10 mg of sodium picosulfate hydrate → 3 packets each |
| 6 | 12 g of citrate hydrate, 3.5 g of magnesium oxide, and 10 mg of sodium picosulfate hydrate → 2 to 3 bottles each |
| 7 | 102.86 mg of anhydrous magnesium sulfate, 201.07 mg of potassium sulfate, 11.43 mg of simethicone, and 1125 mg of anhydrous sodium sulfate → 28 tablets each |
| 8 | 54.6 g of D-sorbitol, 150 g of polyethylene glycol 3350, and 10 mg of sodium picosulfate hydrate → 1 bottle |
| 9 | 1.6 g of anhydrous magnesium sulfate, 3.13 g of potassium sulfate, and 17.5 g of anhydrous sodium sulfate → 2 packets each |
| 10 | 1.6 g of anhydrous magnesium sulfate, 3.13 g of potassium sulfate, and 17.5 g of anhydrous sodium sulfate → 2 bottles each |
| 11 | 2.35 g of ascorbic acid, 2.95 g of sodium ascorbate, 0.50 g of polyethylene glycol 3350, 0.5075 g of potassium chloride, 1.3455 g of sodium chloride, and 3.75 g of anhydrous sodium sulfate → 4 packets each |
| 12 | 29.5 g of polyethylene glycol 3350, 0.37 g of potassium chloride, 0.84 g of sodium hydrogen oxychloride, 0.73 g of |
| | sodium chloride, and 2.85 g of anhydrous sodium sulfate → 8 packets each |
| 13 | 52.5 g of polyethylene glycol 3350, 0.185 g of potassium chloride, 0.715 g of sodium bicarbonate, and 1.4 g of sodium chloride → 8 packets each |
| 14 | 36.67 g of D-sorbitol, 5 g of ascorbic acid, 0.7 g of sodium bicarbonate, and 0.3 g of potassium bicarbonate → 3 packets each |

Of course, the above-mentioned compositions may further comprise the excipients/additives described above.

In an embodiment, the present disclosure provides a bowel cleansing composition comprising:
one or more sugar alcohols selected from xylitol, sorbitol, glycerol, erythritol, threitol, arabitol, ribitol, mannitol, galactitol, fucitol, iditol, inositol, volemitol, isomalt, maltitol, lactitol, maltotriitol, maltotegratol, and polyglycitol as a first effective ingredient;
ascorbic acid or a mixture of ascorbic acid and ascorbate salts as a second effective ingredient; and
a water-soluble solvent,
wherein the content of the first effective ingredient is from about 10 g/L to about 500 g/L, preferably from about 30 g/L to about 300 g/L, more preferably from about 50 g/L to about 100 g/L, and most preferably from about 70 g/L to about 90 g/L based on the total composition,
the content of the second effective ingredient is from about 1 g/L to about 300 g/L, preferably from about 3 g/L to about 100 g/L, more preferably from about 5 g/L to about 50 g/L, and most preferably from about 10 g/L to about 15 g/L based on the total composition,
the content of the water-soluble solvent is from about 1.0 to about 3.0 L, and preferably from about 1.0 to about 1.5 L.

In this case, the bowel cleansing composition may further comprise sodium bicarbonate and/or potassium bicarbonate as a third effective ingredient, and
the content of the third effective ingredient is from about 0.5 g/L to about 5 g/L, preferably from about 1 g/L to about 3 g/L, more preferably from about 1.5 g/L to about 2.5 g/L based on the total composition.

The bowel cleansing composition may further comprise an appropriate amount of a flavoring agent, for example, the flavoring agent may be from about 1.5 g/L to about 3.0 g/L based on the total composition.

In an embodiment, the bowel cleansing composition may comprise
about 110 g of D-sorbitol;
about 15 g of ascorbic acid;
about 2.1 g of sodium bicarbonate;
about 0.9 g of potassium bicarbonate; and
about 1.0 L to 3.0 L of water.

The content (g) of each of the ingredients may be the content of the first part described above, i.e., the effective ingredient. In this case, the water may be administered in the form of a solution in which the ingredients are dissolved, or may be administered alone.

In another embodiment, the composition may comprise from about 1.0 L to about 1.5 L of water, or for the examination, the total dosage taken on the day of and the day before the examination may be from about 1.0 L to about 1.5 L, provided that the total dosage taken on the day of the examination is about 500 mL or less.

In another embodiment, the composition may comprise from about 1.28 L, about 1.35 L, or about 1.44 L of water, or for the examination, the total dosage taken on the day of and the day before the examination is from about 1.28 L, about 1.35 L or about 1.44 L, and the total dosage taken on the day of the examination is about 360 mL or about 500 mL. In this case, the term "dosage" refers to a dosage when the composition is administered in the form of a liquid formulation, for example, a total dosage of the solution when the liquid formulation is in the form of a solution.

In a more specific embodiment, the composition, packaging method, and dosing regimen of the first part of the bowel cleansing composition according to the present disclosure may be as follows.

**[Table 5]**

| Ingredients | Total amount (g) | Packet | Weight per one packet(g) |
|---|---|---|---|
| D-sorbitol | 110 | 3 | 36.67 |
| Ascorbic acid | 15 | 3 | 5.00 |

The composition may further comprise the following excipients.

**[Table 6]**

| Ingredients | Total amount (g) | Packet | Weight per one packet(g) |
|---|---|---|---|
| Sodium bicarbonate | 2.1 | 3 | 0.70 |
| Potassium bicarbonate | 0.9 | 3 | 0.30 |

The composition may further comprise the following excipients.

**[Table 7]**

| Ingredients | Total amount (g) | Packet | Weight per one packet(g) |
|---|---|---|---|
| Blue grape flavor powder | 2.4 | 3 | 0.80 |

The composition may be dissolved in water, et. And taken in the form of preparation solution at the following contents.

**[Table 8]**

| Ingredients | Total amount | Packet | An amount of the preparation solution per one packet |
|---|---|---|---|
| Preparation solution | 960 mL | 3 | 320 mL |

The composition may be taken in divided doses into 960 ml of total preparation solution and 480 ml of additional water in a ratio of "day before examination: day of examination 2:1". For example, specific dosing regimens are as follows: (In the following example, the composition (e.g., first part) packaged in one packet is a powder, and when diluted and dissolved in water to prepare a preparation solution, the content of the preparation solution is substantially the same as the content of water in which it is dissolved. Therefore, in the following example, the content of water dissolving the ingredients in one packet is substantially the same as the content in the preparation solution. Hereafter, in an embodiment of the present disclosure, where a powder is used as the first part, the same applies).
1) A preparation solution obtained by diluting one packet well in 320 mL of water the day before the examination is taken and then one packet diluted with the same way is again taken. Then another 320 mL of water is drunk. If your lips are dry or you feel very thirsty, an appropriate amount of additional water may be drunk. (Total 960 mL)
2) On the day of the examination, one packet diluted in 320 mL of water is taken and then an additional 160 mL of water is drunk. (Total 480 mL)

Alternatively, it may be taken as follows.
1) On the evening of the day before the colonoscopy, 720 mL of the preparation solution is taken and then 360 mL of water is drunk. (Total 1080 mL)
2) On the morning on the day of the colonoscopy, 240 mL of the preparation solution is taken and then 120 mL of water is drunk. (Total 360 mL)

Alternatively,
1) On the evening of the day before the colonoscopy, 320 mL of the preparation solution is taken and an additional 130 mL of water is drunk, and then 320 mL of the preparation solution in the same manner is taken and then an additional 130 mL of water is drunk. (Total 900 mL)
2) on the morning on the day of the colonoscopy, 320 mL of the preparation solution is taken and then 130 mL of water (for a total of 450 mL) is drunk

Alternatively
1) On the evening of the day before the colonoscopy, 320 mL of the preparation solution is taken and then 320 mL of the preparation solution is again taken in the same manner. Then another 160 mL of water is drunk. (Total 800 mL)
2) On the morning on the day of the colonoscopy, 320 mL of the preparation solution is taken and 160 mL of water is drunk (Total 480 mL)

In an embodiment, the present disclosure provides a bowel cleansing composition comprising
polyethyleneglycol as a first effective ingredient;
ascorbic acid or a mixture of ascorbic acid and ascorbate salts as a second effective ingredient; and
water-soluble solvent,
wherein the content of the first effective ingredient is from about 10 g/L to about 500 g/L, preferably from about 30 g/L to about 300 g/L, and more preferably from about 50 g/L to about 200 g/L based on the total composition,
the content of the second effective ingredient is from about 1 g/L to about 300 g/L, preferably from about 3 g/L to about 100 g/L, and more preferably from about 5 g/L to about 60 g/L based on the total composition,
the content of the water-soluble solvent is from about 1.0 to about 3.0 L.

The bowel cleansing composition may further comprise as the third effective ingredient one or more selected from the group consisting of sodium chloride, potassium chloride, and sodium sulfate, and may include, for example, all three of the foregoing. In this case, the content of the third effective ingredient may be from about 0.5 g/L to about 50 g/L, preferably from about 3 g/L to about 30 g/L, and more preferably from about 5 g/L to about 25 g/L based on the total composition.

The polyethylene glycol may have an average molecular weight of 3000 to 4000 Da, preferably polyethylene glycol 3350.

In an embodiment the bowel cleansing composition may comprise
about 200 g of polyethylene glycol 3350;
about 9.4 g of ascorbic acid;
about 11.8 g of sodium ascorbate;
about 15 g of anhydrous sodium sulfate;
about 5.382 g of sodium chloride;
about 2.030 g of potassium chloride; and
about 1.0 to about 3.0 L of water.

The content (g) of each of the ingredients may be the content of the first part described above, i.e., the effective ingredient. In this case, the water may be administered in the form of a solution in which the ingredients are dissolved, or may be administered alone.

In a more specific embodiment, the composition may comprise about 3.0 L of water, or for the examination, the total dosage taken on the day of and the day before the examination may be about 3.0 L, and the total dosage taken on the day of the examination may be about 600 mL.

In a more specific embodiment, the composition, packaging method, and dosing regimen of the first part of the bowel cleansing composition according to the present disclosure may be as follows.

**[Table 9]**

| Effective ingredient | Content (g) | Unit | Total unit | Total content |
|---|---|---|---|---|
| Polyethylene glycol 3350 | 50 | 1 packet | 4 | 200.000 |
| Anhydrous sodium sulfate | 3.75 | 1 packet | 4 | 15.000 |
| Ascorbic acid | 2.35 | 1 packet | 4 | 9.400 |
| Sodium ascorbate | 2.95 | 1 packet | 4 | 11.800 |
| Sodium chloride | 1.3455 | 1 packet | 4 | 5.382 |
| Potassium chloride | 0.5075 | 1 packet | 4 | 2.030 |

1) On the evening of the day before the colonoscopy, 1.6 L of the preparation solution is taken and 800 mL of water is drunk.
2) On the morning on the day of the colonoscopy, 400 mL of the preparation solution is taken and then 200 mL of water is drunk. (Total 600 mL)

In another embodiment the bowel cleansing composition may comprise
about 140 g of polyethylene glycol 3350;
about 7.54 g of ascorbic acid;
about 48.11 g of sodium ascorbate;
about 9 g of anhydrous sodium sulfate;
about 5.2 g of sodium chloride;
about 2.2 g of potassium chloride; and
about 1.0 to about 3.0 L of water.

The content (g) of each of the ingredients may be the content of the first part described above, i.e., the effective ingredient. In this case, the water may be administered in the form of a solution in which the ingredients are dissolved, or may be administered alone.

In a more specific embodiment, the composition may comprise about 2.0 L of water, or for the examination, the total dosage taken on the day of and the day before the examination may be about 2.0 L, and the total dosage taken on the day of the examination may be about 400 mL or about 500 mL.

In a more specific embodiment, the composition, packaging method, and dosing regimen of the first part of the bowel cleansing composition according to the present disclosure may be as follows.

**[Table 10]**

| Effective ingredient | Content (g) | Unit | Total unit | Total content |
|---|---|---|---|---|
| Polyethylene glycol 3350 | 100 | Dose1 | 1 | 100.000 |
| Polyethylene glycol 3350 | 40 | Dose2 | 1 | 40.000 |
| Anhydrous sodium sulfate | 9 | Dose1 | 1 | 9.000 |
| Potassium chloride | 1 | Dose1 | 1 | 1.000 |
| Potassium chloride | 1.2 | Dose2 | 1 | 1.200 |
| Sodium chloride | 2 | Dose1 | 1 | 2.000 |
| Sodium chloride | 3.2 | Dose2 | 1 | 3.200 |
| Sodium ascorbate | 48.11 | Dose2 | 1 | 48.110 |
| Ascorbic acid | 7.54 | Dose2 | 1 | 7.540 |

1) On the evening of the day before the colonoscopy, 750 mL of the preparation solution is taken and 750 mL of water is drunk.
2) On the morning on the day of the colonoscopy, 250 mL of the preparation solution is taken and 250 mL of water is drunk (Total 500 mL).

Alternatively;
1) On the evening of the day before the colonoscopy, 800 mL of the preparation solution is taken and 200 mL of water is drunk.
2) On the morning on the day of the colonoscopy, 200 mL of the preparation solution is taken and 200 mL of water is drunk (Total 400 mL).

In another embodiment the bowel cleansing composition may comprise
about 160 g of polyethylene glycol 3350;
about 40.6 g of ascorbic acid;
about 9.4 g of sodium ascorbate;
about 18 g of anhydrous sodium sulfate;
about 2.7 g of sodium chloride;
about 1 g of potassium chloride; and
about 1.0 to about 3.0 L of water.

The content (g) of each of the ingredients may be the content of the first part described above, i.e., the effective ingredient. In this case, the water may be administered in the form of a solution in which the ingredients are dissolved, or may be administered alone.

In a more specific embodiment, the composition may comprise about 2.0 L of water, or for the examination, the total dosage taken on the day of and the day before the examination may be about 2.0 L, and the total dosage taken on the day of the examination may be about 400 mL.

In a more specific embodiment, the composition, packaging method, and dosing regimen of the first part of the bowel cleansing composition according to the present disclosure may be as follows.

**[Table 11]**

| **Effective ingredient** | **Content (g)** | **Unit** | **Total unit** | **Total content** |
|---|---|---|---|---|
| Ascorbic acid | 20.3 | B agent | 2 | 40.6 |
| Sodium ascorbate | 4.7 | B agent | 2 | 9.4 |
| Polyethylene glycol 3350 | 80 | A agent | 2 | 160 |
| Potassium chloride | 0.5 | A agent | 2 | 1 |
| Sodium chloride | 1.35 | A agent | 2 | 2.7 |
| Anhydrous sodium sulfate | 9 | A agent | 2 | 18 |

1) On the evening of the day before the colonoscopy, 800 mL of the preparation solution is taken and 200 mL of water is drunk.
2) On the morning on the day of the colonoscopy, 200 mL of the preparation solution is taken and 200 mL of water is drunk (Total 400 mL).

In an embodiment, the present disclosure provides, based on the total composition, a bowel cleansing composition comprising
about 0.001 g/L to about 0.1 g/L, and preferably about 0.005 g/L to about 0.05 g/L of sodium picosulfate or a hydrate thereof;
about 0.5 g/L to about 50 g/L, and preferably 1 g/L to about 10 g/L of magnesium oxide;
about 1 g/L to about 50 g/L, and preferably 5 g/L to about 30 g/L of citric acid; and
about 1.0 to 3.0 L of water-soluble solvent.

In an embodiment, the bowel cleansing composition may comprise
about 0.03 g of sodium picosulfate hydrate;
about 10.5 g of magnesium oxide;
about 36 g of citric acid; and
about 1.0 to about 3.0 L of water.

The content (g) of each of the ingredients may be the content of the first part described above, i.e., the effective ingredient. In this case, the water may be administered in the form of a solution in which the ingredients are dissolved, or may be administered alone.

In a more specific embodiment, the composition may comprise about 3.0 L of water, or for the examination, a total dose taken on the day of and the day before the examination may be about 3.0 L, and the total dosage taken on the day of the examination may be about 600 mL.

In a more specific embodiment, the composition, packaging method, and dosing regimen of the first part of the bowel cleansing composition according to the present disclosure may be as follows.

**[Table 12]**

| **Effective ingredient** | **Content (g)** | **Unit** | **Total unit** | **Total content** |
|---|---|---|---|---|
| Picosulfate sodium hydrate | 0.01 | 1 packet | 3 | 0.030 |
| Hard magnesium oxide | 3.5 | 1 packet | 3 | 10.500 |
| Citric acid | 12 | 1 packet | 3 | 36.000 |

1) On the evening of the day before the colonoscopy, 240 mL of the preparation solution is taken and 2,160 mL of water is drunk.
2) On the morning on the day of the colonoscopy, 60 mL of the preparation solution is taken and 540 mL of water is drunk (Total 600 mL).

In an embodiment, the present disclosure provides, based on the total composition, a bowel cleansing composition comprising
about 10 g/L to about 500 g/L, preferably about 30 g/L to about 300 g/L, and more preferably about 50 g/L to about 100 g/L of polyethylene glycol;
about 1 g/L to about 300 g/L, preferably about 10 g/L to about 100 g/L, and more preferably about 20 g/L to about 50 g/L of sorbitol;
0.001 g/L to 1 g/L, preferably about 0.002 g/L to about 0.007 g/L of sodium picosulfate or a hydrate thereof; and
about 1.0 to 3.0 L of water-soluble solvent.

In an embodiment, the bowel cleansing composition may comprise
about 75 g/L of polyethylene glycol;
about 27.3 g/L of sorbitol;
about 0.005 g/L of sodium picosulfate or a hydrate thereof; and
about 1.0 to about 3.0 L of water.

The content (g) of each of the ingredients may be the content of the first part described above, i.e., the effective ingredient. In this case, the water may be administered in the form of a solution in which the ingredients are dissolved, or may be administered alone.

In a more specific embodiment, the composition may comprise about 2.0 L of water, or in this case, for the examination, the total dosage taken on the day of and the day before the examination may be about 2.0 L, and the total dosage taken on the day of the examination may be about 400 mL.

In a more specific embodiment, the composition, packaging method, and dosing regimen of the first part of the bowel cleansing composition according to the present disclosure may be as follows.

**[Table 13]**

| **Effective ingredient** | **Content (g)** | **Total unit** | **Total content** |
|---|---|---|---|
| Polyethylene glycol 3350 | 150 | 1 | 150 |
| D-sorbitol | 54.6 | 1 | 54.6 |
| Sodium picosulfate hydrate | 0.01 | 1 | 0.01 |
| The contnent of above ingredients is one contained in about 1 bottle (300 mL). | | | |

1) On the evening of the day before the colonoscopy, 800 mL of the preparation solution is taken and 200 mL of water is drunk.
2) on the morning on the day of the colonoscopy, 200 mL of the preparation solution is taken and 200 mL of water is drunk (Total 400 mL).

In an embodiment, the present disclosure provides, based on the total composition, a bowel cleansing composition comprising
about 5 g/L to about 300 g/L, preferably about 7 g/L to about 50 g/L, and more preferably about 10 g/L to about 30 g/L of sodium sulfate;
about 0.1 g/L to about 50 g/L, preferably about 0.5 g/L to about 10 g/L, and more preferably about 0.7 g/L to about 5 g/L of potassium sulfate;
about 0.1 g/L to about 50 g/L, preferably about 0.5 g/L to about 10 g/L, and more preferably about 0.7 g/L to about 5 g/L of magnesium sulfate;
about 1.0 to 3.0 L of water-soluble solvent.

In an embodiment, the bowel cleansing composition may comprise
about 12.333 g/L of anhydrous sodium sulfate;
about 2.206 g/L of potassium sulfate;
about 1.128 g/L of magnesium sulfate; and
about 1.0 to about 3.0 L of water.

The content (g) of each of the ingredients may be the content of the first part described above, i.e., the effective ingredient. In this case, the water may be administered in the form of a solution in which the ingredients are dissolved, or may be administered alone.

In a more specific embodiment, the composition may comprise about 2.838 L of water, or in this case, for the examination, the total dosage taken on the day of and the day before the examination may be about 2.838 L, and the total dosage taken on the day of the examination may be about 568 mL.

In a more specific embodiment, the composition, packaging method, and dosing regimen of the first part of the bowel cleansing composition according to the present disclosure may be as follows.

**[Table 14]**

| **Effective ingredient** | **Content (g)** | **Unit** | **Total unit** | **Total content** |
|---|---|---|---|---|
| Anhydrous sodium sulfate | 17.5 | Bottle | 2 | 35.000 |
| Potassium sulfate | 3.13 | Bottle | 2 | 6.260 |
| Anhydrous magnesium sulfate | 1.6 | Bottle | 2 | 3.200 |
| Anhydrous magnesium sulfate | 1.6 | Bottle | 2 | 3.200 |
| The content of above ingredients is one contained in about 1 bottle (177 mL). | | | | |

1) On the evening of the day before the colonoscopy, 757 mL of the preparation solution is taken and 1,514 mL of water is drunk.
2) on the morning on the day of the colonoscopy, 189 mL of the preparation solution is taken and 378 mL of water is drunk (Total 568 mL).

### [Advantageous Effects]

For most existing bowel cleansers, first dosage taken the day before the examination and the second dosage taken on the day of the examination are the same, and the total dosage of the preparation solution or the preparation medicine and water on the day of the examination exceed at least 1 liter. The end time for taking bowel cleansers is also 1-2 hours immediately before the examination, which is contrary to the principle of ending taking a dose at 2-3 hours before the examination or at least 3 hours before the sedation endoscopy recommended in existing documents. Ultimately, there is a problem that existing bowel cleansers inevitably reduce the safety and ease of use for subjects.

Some bowel cleansers have different dosages for the evening of the day before and on the day of the examination, so that on the day to the examination you take a smaller amount the preparation solution and water than the day before the examination. However, since they still require approximately 1 liter or more of preparation medicine and water on the day of the examination as well as the day before the examination, which does not address the safety of colonoscopy and the potential risks of combining colonoscopy with gastroscopy described above.

This dosing regimen of drinking a large amount of water up to 1-2 hours prior to the examination cannot completely exclude safety issues related to the risk of aspiration during concurrent gastroscopy or sedation endoscopy.

On the other hand, the bowel cleansing composition according to the present disclosure has a total dosage of 700 mL or less, for example, 200 to 700 mL, preferably 600 mL or less, and more preferably 500 mL or less on the day of the examination, which is very small comparted to the existing dosage, but exhibits a bowel cleaning or cleaning effect to the extent that the effectiveness of colonoscopy may be secured, and at the same time, the safety of the subject can be improved more effectively. Furthermore, due to the small dosage, the dosing time is reduced accordingly, so the interval between examination times may be further secured, and the safety of the examination may be improved by minimizing the amount of preparation solution and water remaining in the stomach and large intestine.

### [Best Mode]

Hereinafter, the present disclosure will be described in more detail through Examples. However, the present disclosure is not limited to the following Examples.

Randomly selected patients were administered each of the following compositions according to the dosing regimens described in each of the Examples and Comparative Examples, and degree of bowel preparation, endoscopy time, bowel residual amount, ease of dosage, side effects (adverse events - nausea, vomiting, dizziness, etc.), willingness to take drug again, etc. were evaluated.

### Example 1

In this Example, the compositions of the ingredients and contents in the Table below were used.

**[Table 15]**

| **Effective ingredient** | **Total content (g)** |
|---|---|
| Polyethylene glycol 3350 | 200.000 |
| Anhydrous sodium sulfate | 15.000 |
| Ascorbic acid | 9.400 |
| Sodium ascorbate | 11.800 |
| Sodium chloride | 5.382 |
| Potassium chloride | 2.030 |
| **Total amount** | **243.612** |

### Dosing regimen

The effective ingredients of the above Table were mixed well with each other by content, an appropriate amount of water was added to dissolve them, and then water was added again to be a total amount of 2 L, and mixed well with each other to prepare a preparation solution. For 2 L of total preparation solution, a total of 1 L of water should be taken.

In each Comparative Example and Example, the instructions were to divide the total preparation solution into the corresponding ratio (%) of the previous day's or the day's preparation solution and take it with water, or to divide the effective ingredients in the above Table into the content corresponding to the corresponding ratio (%), separately prepare the previous day's or the day's preparation solution and then take it with water. The dosing regimen (a ratio % of the preparation solution) for each Comparative Example and Example is as follows.

**[Table 16]**

| | |
|---|---|
| **Comparative Example 1** | **Example 1.** |
| Primary: 50% | Primary: 80% |
| On the day before the colonoscopy, 1 L of the preparation solution was taken and an additional 500 mL of water was drunk. | On the evening of the day before the colonoscopy, 1.6 L of the preparation solution was taken and 800 mL of water was drunk. |
| Secondary: 50% | Secondary: 20% |
| On the morning on the day of the colonoscopy, 1 L of the preparation solution was taken and an additional 500 mL of water was drunk. | On the morning on the day of the colonoscopy, 400 mL of the preparation solution and 200 mL of water was drunk (Total 600 mL) . |

**[Table 17]**

| Classificat ion | | Previous day | | | The day | | | Total | | |
|---|---|---|---|---|---|---|---|---|---|---|
| | | Preparat ion solution | Water | Total | preparat ion solution | Water | Total | preparat ion solution | Water | Total amount |
| Comp. Example 1 | % | 50% | 50% | | 50% | 50% | | 100% | 100% | |
| | ml | 1,000 | 500 | 1,500 | 1,000 | 500 | 1,500 | 2,000 | 1,000 | 3,000 |
| Example 1 | % | 80% | 80% | | 20% | 20% | | 100% | 100% | |
| | ml | 1,600 | 800 | 2,400 | 400 | 200 | 600 | 2,000 | 1,000 | 3,000 |

### Example 2

In this Example, the compositions of the ingredients and contents in the Table below were used.

**[Table 18]**

| **Effective ingredient** | **Total content (g)** |
|---|---|
| Polyethylene glycol 3350 | 140 |
| Anhydrous sodium sulfate | 9 |
| Potassium chloride | 2.2 |
| Sodium chloride | 5.2 |
| Sodium ascorbate | 48.11 |
| Ascorbic acid | 7.54 |
| **Total amount** | **212.05** |

The effective ingredients of the above Table were mixed well with each other by content, an appropriate amount of water was added to dissolve them, and then water was added again to be a total amount of 1 L, and mixed well with each other to prepare a preparation solution. For 1 L of total preparation solution, a total of 1 L of water should be taken.

In each Comparative Example and Example, the instructions were to divide the total preparation solution into the corresponding ratio (%) of the previous day's or the day's preparation solution and take it with water, or to divide the effective ingredients in the above Table into the content corresponding to the corresponding ratio (%), separately prepare the previous day's or the day's preparation solution and then take it with water. The dosing regimen (a ratio % of the preparation solution) for each Comparative Example and Example is as follows.

**[Table 19]**

| | |
|---|---|
| **Comparative Example 2** | **Example 2** |
| Primary: 50% | Primary: 75% |
| On the day before the colonoscopy, 500 mL of the preparation solution was taken and an additional 500 mL of water was drunk. | On the evening of the day before the colonoscopy, 750 mL of the preparation solution was taken and 750 mL of water was drunk. |
| Secondary: 50% | Secondary: 25% |
| On the morning on the day of the colonoscopy, 500 mL of the preparation solution was taken and an additional 500 mL of water was drunk. | On the morning on the day of the colonoscopy, 250 mL of the preparation solution was taken and 250 mL of water was drunk (Total 500 mL). |

**[Table 20]**

| Classification | | Previous day | | | The day | | | Total | | |
|---|---|---|---|---|---|---|---|---|---|---|
| | | preparat ion solution | Water | Total | preparat ion solution | Water | Total | preparat ion solution | Water | Total amount |
| Comp. example 2 | % | 50% | 50% | | 50% | 50% | | 100% | 100% | |
| | Volume | 500 | 500 | 1, 000 | 500 | 500 | 1,000 | 1, 000 | 1,000 | 2,000 |
| Example 2 | % | 75% | 75% | | 25% | 25% | | 100% | 100% | |
| | Volume | 750 | 750 | 1,500 | 250 | 250 | 500 | 1, 000 | 1,000 | 2,000 |

### Example 3

n this Example, the compositions of the ingredients and contents in the Table below were used. The relevant composition was prepared by mixing each effective ingredient.

**[Table 21]**

| **Effective ingredient** | **Total content (g)** |
|---|---|
| D-sorbitol | 110 |
| Ascorbic acid | 15 |
| Sodium bicarbonate | 2.1 |
| Potassium bicarbonate | 0.9 |
| Flavor Powder | 2.4 |
| **Total amount** | **130.4** |

### Dosing regimen

The effective ingredients of the above table were mixed well with each other by content, an appropriate amount of water was added to dissolve them, and then water was added again to be a total amount of 960 mL, and mixed well with each other to prepare a preparation solution. For 960 mL of total preparation solution, a total of 480 mL of water should be taken.

In each Comparative Example and Example, the instructions were to divide the total preparation solution into the corresponding ratio (%) of the previous day's or the day's preparation solution and take it with water, or to divide the effective ingredients in the above Table into the content corresponding to the corresponding ratio (%), separately prepare the previous day's or the day's preparation solution and then take it with water. The dosing regimen (a ratio % of the preparation solution) for each Comparative Example and Example is as follows.

**[Table 22]**

| | |
|---|---|
| **Comparative Example 3** | **Example 3** |
| Primary: 50% | Primary: 75% |
| On the day before the colonoscopy, 480 mL of the preparation solution was taken and an additional 240 mL of water was drunk. | On the evening of the day before the colonoscopy, 720 mL of the preparation solution was taken and 360 mL of water was drunk. |
| Secondary: 50% | Secondary: about 25% |
| On the morning on the day of the colonoscopy, 480 mL of the preparation solution was taken and an additional 240 mL of water was drunk. | On the morning on the day of the colonoscopy, 240 mL of the preparation solution was taken and 120 mL of water was drunk (Total 360 mL). |

**[Table 23]**

| Classificat ion | | Previous day | | | The day | | | Total | | |
|---|---|---|---|---|---|---|---|---|---|---|
| | | Preparat ion solution | Water | Total | Preparat ion solution | Water | Total | Preparat ion solution | Water | Total amount |
| Comp. example 3 | % | 50% | 50% | | 50% | 50% | | 100% | 100% | |
| | Volu me | 480 | 240 | 720 | 480 | 240 | 720 | 960 | 480 | 1,440 |
| Example 3 | % | 75% | 75% | | 25% | 25% | | 100% | 100% | |
| | Volu me | 720 | 360 | 1080 | 240 | 120 | 360 | 960 | 480 | 1,440 |

### Example 4

In this Example, the compositions of the ingredients and contents in the Table below were used.

**[Table 24]**

| **Effective ingredient** | **Total content (g)** |
|---|---|
| Sodium picosulfate hydrate | 0.03 |
| Hard magnesium oxide | 10.5 |
| Citric acid | 36 |
| **Total amount** | **46.53** |

### Dosing regimen

The effective ingredients of the above table were mixed well with each other by content, an appropriate amount of water was added to dissolve them, and then water was added again to be a total amount of 300 mL, and mixed well with each other to prepare a preparation solution. For 300 mL of total preparation solution, a total of 2700 mL of water should be taken.

In each Comparative Example and Example, the total preparation solution was taken by dividing it into the corresponding ratio (%) of the previous day's or the day's preparation solution, or the effective ingredients in the above Table was divided into the content corresponding to the corresponding ratio (%), the previous day's or the day's preparation solution was separately prepared and then taken.

The dosing regimen (a ratio % of the preparation solution) of each Example is as follows.

**[Table 25]**

| | |
|---|---|
| **Comparative Example 4** | **Example 4** |
| Primary: 50% | Primary: 80% |
| On the day before the colonoscopy, 150 mL of the preparation solution was taken and an additional 1,350 mL of water was drunk. | On the evening of the day before the colonoscopy, 240 mL of the preparation solution was taken and 2,160 mL of water was drunk. |
| Secondary: 50% | Secondary: 20% |
| On the morning on the day of the colonoscopy, 150 mL of the preparation solution was taken and an additional 1,350 mL of water was drunk. | On the morning on the day of the colonoscopy, 60 mL of the preparation solution was taken and 540 mL of water was drunk (Total 600 mL). |

**[Table 26]**

| Classification (actual) | | Previous day | | | The day | | | Total | | |
|---|---|---|---|---|---|---|---|---|---|---|
| | | Prepar ation soluti on | Water | Total | Prepar ation soluti on | Water | Total | Prepar ation soluti on | Water | Total amount |
| Comp. example 4 | % | 50% | 50% | | 50% | 50% | | 100% | 100% | |
| | Volume | 150 | 1,350 | 1,500 | 150 | 1,350 | 1,500 | 300 | 2,700 | 3,000 |
| Example 4 | % | 80% | 80% | | 20% | 20% | | 100% | 100% | |
| | Volume | 240 | 2,160 | 2,400 | 60 | 540 | 600 | 300 | 2,700 | 3,000 |

### Example 5

In this Example, the compositions of the ingredients and contents in the Table below were used.

**[Table 27]**

| **Effective ingredient** | **Total content (g)** |
|---|---|
| Anhydrous sodium sulfate | 35 |
| Potassium sulfate | 6.26 |
| Anhydrous magnesium sulfate | 3.2 |
| **Total amount** | **44.46** |

### Dosing regimen

The effective ingredients of the above Table were mixed well with each other by content, an appropriate amount of water was added to dissolve them, and then water was added again to be a total amount of 946 mL, and mixed well with each other to prepare a preparation solution. For 946 mL of total preparation solution, a total of 1892 mL of water should be taken.

In each Comparative Example and Example, the total preparation solution was taken by dividing it into the corresponding ratio (%) of the previous day's or the day's preparation solution, or the effective ingredients in the above Table was divided into the content corresponding to the corresponding ratio (%), the previous day's or the day's preparation solution was separately prepared and then taken.

The dosing regimen (a ratio % of the preparation solution) of each Example is as follows.

**[Table 28]**

| | |
|---|---|
| **Comparative Example 5** | **Example 5** |
| Primary: 50% | Primary: 80% |
| On the day before the colonoscopy, 473 mL of the preparation solution was taken and an additional 946 mL of water was drunk. | On the evening of the day before the colonoscopy, 757 mL of the preparation solution was taken and 1,514 mL of water was drunk. |
| Secondary: 50% | Secondary: 20% |
| | On the morning on the day of the colonoscopy, 189 mL of the |
| On the morning on the day of the colonoscopy, 473 mL of the preparation solution was taken and an additional 946 mL of water was drunk. | preparation solution was taken and 378 mL of water was drunk (Total 568 mL). |

**[Table 29]**

| Classificat ion | | Previous day | | | The day | | | Total | | |
|---|---|---|---|---|---|---|---|---|---|---|
| | | Preparat ion solution | Water | Total | Preparat ion solution | Water | Total | Preparat ion solution | Water | Total amount |
| Comp. Example 5 | % | 50% | 50% | | 50% | 50% | | 100% | 100% | |
| | Volu me | 473 | 946 | 1,419 | 473 | 946 | 1,419 | 946 | 1,892 | 2,838 |
| Example 5 | % | 80% | 80% | | 20% | 20% | | 100% | 100% | |
| | Volu me | 757 | 1,514 | 2,270 | 189 | 378 | 568 | 946 | 1,892 | 2,838 |

### Results of Examples 1 to 5

Randomly selected patients were administered each of the following compositions according to the dosing regimens described in each of the Examples and Comparative Examples, and degree of bowel preparation, examination time, stomach/bowel residual amount, dosing time, frequency of morning bowel movements, dose completion rate, and side effects (adverse cases - nausea, vomiting, dizziness, etc.), willingness to take the drug again, etc. were evaluated. Patients above were healthy adults who were scheduled for routine outpatient colonoscopy, had no prior adverse reactions to other commercially available bowel preparation agents, and patients having serious medical problems, heart disease, diabetes, or any other potentially serious condition were excluded.

Among the evaluation items, degree of bowel preparation, residual amount, and examination time (minutes) were evaluated by the clinician who performed the endoscopy.

### Bowel preparation success rate

The degree of bowel preparation was confirmed by colonoscopy. To objectively evaluate the bowel preparation effect, the grade of bowel cleaning effect was evaluated based on the Harefield Cleansing Scale, which categorizes the degree of bowel preparation of each subject into five phases, was evaluated.

**[Table 30]**

| Grade | Criteria |
|---|---|
| Excellent (1 point) | bowel empty and clean |
| Good (2 point) | presence of clear liquid in the digestive gut |
| Fair (3 point) | presence of brown liquid or small amounts of semisolid residual stool, fully removable by suction or displaceable, thus allowing a complete visualization of the underlying mucosa |
| Poor (4 point) | presence of semisolid stool, only partially removable with a risk of incomplete underlying mucosal visualization |
| Fail (5 point) | presence of semisolid or solid stool, colonoscopy incomplete or had to be stopped |

The above five scores were categorized into grades from A to D as follows to evaluate the degree of bowel preparation. Specifically, according to the Harefield cleanliness scale, grades of A and B were considered successful bowel cleansing and grades of C and D were considered unsuccessful bowel cleansing.

### <Evaluation of overall degree of colon preparation>

Grade A: All segments scored 1 or 2
Grade B: 1 segment scored 3
Grade C: 1 segment scored 4
Grade D: 1 segment scored 5

### Residual amount

Gastric residual amount and bowel residual amount were measured by summing the residual water in the stomach through the suction channel in the scope during endoscopy.

### Examination times

The time from start to completion of colonoscopy was recorded.

Among the evaluation items, dosing time (minutes), frequency of morning bowel movements (times), dose completion rate (%), adverse events (%), and willingness to take the drug again (%) were evaluated through a survey of the subjects (patients). Among them, dose completion rate, adverse events, and willingness to take the drug again were calculated with a rate of the number of subject corresponding to relevant items over whole subjects.

The results are shown in Table 31.

**[Table 31]**

| Classification | | Example 1 | | Example 2 | | Example 3 | | Example 4 | | Example 5 | |
|---|---|---|---|---|---|---|---|---|---|---|---|
| | | Compari son | Run | Compari son | Run | Compari son | Run | Compari son | Run | Compari son | Run |
| Total solution dosage for the day (ml; preparation solution and water) | | 1500 | 600 | 1000 | 500 | 720 | 360 | 1500 | 600 | 1419 | 568 |
| N | | 49 | 49 | 47 | 48 | 49 | 51 | 45 | 45 | 42 | 42 |
| Bowel prepara tion success rate | N | 43 | 45 | 43 | 45 | 46 | 47 | 41 | 42 | 39 | 38 |
| | % | 87.8% | 91. 8% | 91.5% | 93. 8% | 93.9% | 92.2 % | 91.1% | 93. 3% | 92.9% | 90. 5% |
| Examina tion time | (minut es) | 16.6 | 12. 5 | 14.5 | 12. 1 | 13.5 | 9.8 | 14.4 | 13. 2 | 15.1 | 13. 2 |
| Residua l amount of stomach | (mL) | 27.2 | 8.6 | 19.2 | 7.9 | 15.5 | 5.1 | 30.3 | 9.7 | 28.9 | 8.6 |
| Residua 1 amount of the bowel | (Point ) | 23.27 | 15. 71 | 20.43 | 14. 79 | 17.14 | 12.7 5 | 21.78 | 16. 00 | 21.90 | 17. 62 |
| Dosing time (successful person, min) | | 107 | 41 | 68 | 22 | 34 | 18 | 105 | 36 | 111 | 35 |
| Frequency of morning bowel movements (times) | | 8.7 | 3.9 | 7.2 | 3.8 | 4.5 | 3.1 | 6.2 | 4.1 | 5.1 | 3.9 |
| Dose completion rate (%) | | 91.8 | 98. 0 | 95.7 | 100 | 98 | 100 | 95.6 | 97. 8 | 92.9 | 95. 2 |
| Adverse event (%) | Nausea | 22.4 | 12. 2 | 19.1 | 8.3 | 10.2 | 3.9 | 22.2 | 13. 3 | 21.4 | 9.5 |
| | Vomiti ng | 10.2 | 4.1 | 8.5 | 4.2 | 2 | - | 8.9 | 4.4 | 11.9 | 7.1 |
| | Urgent stool | 26.5 | 10. 2 | 19.1 | 6.3 | 10.2 | 2 | 22.2 | 8.9 | 19 | 9.5 |
| willing ness to take the drug again | N | 23 | 39 | 28 | 42 | 47 | 51 | 20 | 28 | 18 | 30 |
| | % | 46.9% | 79. 6% | 59.6% | 87. 5% | 95.9% | 100. 0% | 44.4% | 62. 2% | 42.9% | 71. 4% |

Examples 1 to 5, all of which had a total daily dosage of 700 ml or less of a preparation solution and water, were superior or equivalent in terms of bowel preparation success rate compared to the Comparison Examples. In terms of gastric residual amount/bowel residual amount, which is an indicator of safety, the residual amount was significantly reduced compared to each Comparison Example, and in particular, the gastric residual amount was reduced by less than half compared to the comparison example. A significant number of colonoscopy patients also undergo gastroscopy, but fasting for at least 8 hours, including water, is recommended during gastroscopy, which is directly contradictory to the method of taking bowel cleansers for colonoscopy, and thus in terms of improving the subject's safety, gastric residual amount is also an important evaluation criterion. When taking the example composition according to the present disclosure, the gastric residual amount was dramatically improved, and the safety of the subject was very effectively improved.

In addition, the time required for endoscopies was reduced compared to the Comparative Example, and this is because the bowel environment is suitable for colonoscopy, so the time to reach the cecum and the recovery time during endoscopy were shortened. The ease of examination also increased, making it easier to secure a view for endoscopy and polyp identification, etc. due to the degree of bowel preparation and reduction of residual fluid.

The patient's dosing time was significantly reduced to less than half compared to the Comparative Example, and the number of morning bowel movements also decreased, increasing the patient's convenience of taking the drug and also increasing the dose completion rate.

Adverse events such as nausea, vomiting, and urgent stool were very significantly reduced compared to the Comparative Example.

The patient's willingness to take the drug again also increased significantly, and in particular, in Example 3, the willingness to take the drug again was 100%.

### Examples 6 to 16

Similar to Examples 1 to 5, the composition of each effective ingredient in the Table below was administered in a ratio of 80:20 to 75:25 on the previous day vs the day (provided that the total dosage amount of the preparation solution and water for the day was 700 mL, 600 mL, or 500 mL or less). Examples 6 to 16 are the composition of a product that has already been approved by the Korean Ministry of Food and Drug Safety as a bowel preparation agent, and the package of the relevant product was used as is, and like Examples 1 to 5, the total preparation solution was taken by dividing it into the corresponding ratio (%) of the previous day's or the day's preparation solution, or each effective ingredient was divided into the content corresponding to the corresponding ratio (%), the previous day's or the day's preparation solution was separately prepared and then taken. The dosing regimen (a ratio % of the preparation solution) of each Example is as follows.

**[Table 32]**

| **Examples** | **Ingredients** | **Content (g)** | **Total preparation solution (ml)** | **Total water (ml)** |
|---|---|---|---|---|
| Example 6 | D-sorbitol | 75.80 | 460 | 920 |
| | Ascorbic acid | 10 | | |
| | Sodium picosulfate hydrate | 0.02 | | |
| Example 7 | Ascorbic acid | 40.6 | 1000 | 1000 |
| | Sodium ascorbate | 9.4 | | |
| | Polyethylene glycol 3350 | 160 | | |
| | Potassium chloride | 1 | | |
| | Sodium chloride | 2.7 | | |
| | Anhydrous sodium sulfate | 18 | | |
| Example 8 | Anhydrous sodium hydrogen phosphate | 12.736 | (Tablet form) | about 2000 |
| | Sodium dihydrogen phosphate monohydrate | 35.264 | | |
| Example 9-1 | Citrate hydrate | 24 | 340 | 2000 |
| | Magnesium oxide | 7.0 | | |
| | Sodium picosulfate hydrate | 0.02 | | |
| Example 9-2 | Citrate hydrate | 36 | 500 | 3000 |
| | Magnesium oxide | 10.5 | | |
| | Sodium picosulfate hydrate | 0.03 | | |
| Example 10 | Ahydrous magnesium sulfate | 2.88008 | (Tablet form) | 2,550 |
| | Potassium sulfate | 5.62996 | | |
| | Simethicon | 0.32004 | | |
| | Anhydrous sodium sulfate | 31.5 | | |
| Example 11 | D-sorbitol | 54.6 | 1000 | 1000 |
| | Polyethylene glycol 3350 | 150 | | |
| | Sodium picosulfate hydrate | 0.01 | | |
| Example 12 | Anhydrous magnesium sulfate | 3.2 | 946 | 1892 |
| | Potassium sulfate | 6.26 | | |
| | Anhydrous sodium sulfate | 35 | | |
| Example 13 | Anhydrous magnesium sulfate | 3.2 | 946 | 1892 |
| | Potassium sulfate | 6.26 | | |
| | Anhydrous sodium sulfate | 35 | | |
| Example 14 | Ascorbic acid | 9.4 | 2000 | 1000 |
| | Sodium ascorbate | 11.8 | | |
| | Polyethylene glycol 3350 | 200 | | |
| | Potassium chloride | 2.03 | | |
| | Sodium chloride | 5.382 | | |
| | Anhydrous sodium sulfate | 15 | | |
| Example 15 | Polyethylene glycol 3350 | 236 | 4000 | - |
| | Potassium chloride | 2.96 | | |
| | Sodium bicarbonate | 6.72 | | |
| | Sodium chloride | 5.84 | | |
| | Anhydrous sodium sulfate | 22.8 | | |
| Example 16 | Polyethylene glycol 3350 | 420 | 4000 | |
| | Potassium chloride | 1.48 | | |
| | Sodium bicarbonate | 5.72 | | |
| | sodium chloride | 11.2 | | |

### Example 17

Compositions were prepared according to the ingredients and contents in the Table below (see example 3).

**[Table 33]**

| **Effective ingredient** | **Total content (g)** |
|---|---|
| D-sorbitol | 110.0 |
| Ascorbic acid | 15.0 |
| Sodium bicarbonate | 2.1 |
| Potassium bicarbonate | 0.9 |
| Flavor Powder | 2.4 |
| **Total amount** | **130.4** |

### (1) Example 17-1

### Dosing regimen

The effective ingredients of the above Table were mixed well with each other by content, an appropriate amount of water was added to dissolve them, and then water was added again to be a total amount of 960 mL, and mixed well with each other to prepare a preparation solution. For 960 mL of total preparation solution, a total of 390 mL of water should be taken.

In each Comparative Example and Example, the instructions were to divide the effective ingredients in the above Table into the content corresponding to the corresponding ratio (%), separately prepare the previous day's or the day's preparation solution and then take it with water.

In the Comparative Example, the composition was divided into two packages and each package was taken the previous day and the day. In the Example, a total of three packaging units were provided, 2 packets on the previous day and 1 packet on the morning of the day, that is, each packaging unit in the Example contained 36.67 g of D-sorbitol, 5 g of ascorbic acid, 0.7 g of sodium bicarbonate, 0.3 g of potassium bicarbonate, and 0.8 g of fragrance powder. It was recommended that the subjects take the following doses: The packaging unit taken on the evening of the day before the examination was labeled as A agent and the packaging unit taken on the day of the examination were labeled as B agent.
(1) On 8 p.m. evening of the day before the examination, a solution obtained by diluting 1 packet of A agent well in 320 mL of water was drunk over 10 minutes, and then an additional 130 mL of water was drunk. Then 1 packet again in the same way (320 mL of water) was diluted and was taken over 10 minutes, and then additional 130 mL of water was drunk.
(2) On the early morning or the morning on the day of the examination, 1 packet of B agent was diluted in 320 mL of water and was taken over 10 minutes. Hereinafter, additional 130 mL of water was drunk. For the safety during the examination, it is good to finish taking the dose up to 2 hours before the examination starts.

In the Comparative Example, each of the four packaging agents was prepared. That is, this means that each packaging unit contained 27.5 g of D-sorbitol, 3.75 g of ascorbic acid, 0.525 g of sodium bicarbonate, 0.225 g of potassium bicarbonate, and 0.6 g of flavor powder. It was recommended that the subjects take the following doses: The packaging unit taken on the evening of the day before the examination was labeled as A agent and the packaging unit taken on the day of the examination were labeled as B agent.
(1) On 8 p.m. evening of the day before the examination, a solution obtained by diluting 1 packet of A agent well in 240 mL of water was drunk over 10 minutes, and then an additional 97.5 mL of water was drunk. Then 1 packet again in the same way (240 mL of water) was diluted and was taken over 10 minutes, and then another 97.5 mL of water was drunk.
(2) On the early morning or morning on the day of the examination, a solution obtained by diluting 1 packet of B agent well in 240 mL of water was drunk over 10 minutes and an additional 97.5 mL of water was drunk. Then 1 packet again in the same way (240 mL of water) was diluted and was taken over 10 minutes, and then additional 97.5 mL of water was drunk. For the safety during the examination, it is good to finish taking the dose up to 2 hours before the examination starts.

The dosing regimen of each Comparative Example and Example is as follows.

**[Table 34]**

| | |
|---|---|
| **Comparative Example 17-1** | **Example 17-1** |
| Primary: 50% | Primary: 67% |
| On the day before the colonoscopy, 480 mL of the preparation solution was taken and then an additional 195 mL of water was drunk. | On the evening of the day before the colonoscopy, 640 mL of the preparation solution was taken and 260 mL of water was drunk. |
| Secondary: 50% | Secondary: 33% |
| On the morning on the day of the colonoscopy, 480 mL of the preparation solution was taken and an additional 195 mL of water was drunk. | On the morning on the day of the colonoscopy, 320 mL of the preparation solution was taken and 130 mL of water was drunk. |

**[Table 35]**

| Classifi cation | | Previous day | | | The day | | | Total | | |
|---|---|---|---|---|---|---|---|---|---|---|
| | | Prepara tion solutio n | Water | Total | Preparat ion solution | Water | Total | Preparat ion solution | Water | Total amount |
| Comp. Exampl e 17-1 | % | 50% | 50% | | 50% | 50% | | 100% | 100% | |
| | ml | 480 | 195 | 675 | 480 | 195 | 675 | 960 | 390 | 1,350 |
| Exampl e 17-1 | % | 67% | 67% | | 33% | 33% | | 100% | 100% | |
| | ml | 640 | 260 | 900 | 320 | 130 | 450 | 960 | 390 | 1, 350 |

### (2) Example 17-2

### Dosing regimen

The effective ingredients of the above Table were mixed well with each other by content, an appropriate amount of water was added to dissolve them, and then water was added again to be a total amount of 960 mL, and mixed well with each other to prepare a preparation solution. For 960 mL of total preparation solution, a total of 320 mL of water should be taken.

In each Comparative Example and Example, the instructions were to divide the effective ingredients in the above Table into the content corresponding to the corresponding ratio (%), separately prepare the previous day's or the day's preparation solution and then take it with water.

The dosing regimen of each Comparative Example and Example is as follows.

**[Table 36]**

| **Comparative example 17-2** | **Example 17-2** |
|---|---|
| Primary: 50% | Primary: 63% |
| On the day before the colonoscopy, 480 mL of the | On the evening of the day before the colonoscopy, 640 mL of the |
| preparation solution was taken and then an additional 160 mL of water was taken. | preparation solution was taken and 160 mL of water was drunk. |
| | Secondary: 37% |
| Secondary: 50% | On the morning on the day of the colonoscopy, 320 mL of the preparation solution was taken and 160 mL of water was drunk. (Total 480 mL) |
| On the morning on the day of the colonoscopy, 480 mL of the preparation solution was taken and an additional 160 mL of water was drunk. | |

**[Table 37]**

| Classificat ion | | Previous day | | | The day | | | Total | | |
|---|---|---|---|---|---|---|---|---|---|---|
| | | Preparat ion solution | Water | Total | Preparat ion solution | Water | Total | Preparat ion solution | Water | Total amount |
| Comp. Example 17-2 | % | 50% | 50% | | 50% | 50% | | 100% | 100% | |
| | ml | 480 | 160 | 640 | 480 | 160 | 640 | 960 | 320 | 1,280 |
| Example 17-2 | % | 67% | 50% | | 33% | 50% | | 100% | 100% | |
| | ml | 640 | 160 | 800 | 320 | 160 | 480 | 960 | 320 | 1,280 |

### Example 18

In this Example, the compositions of the ingredients and contents in the Table below were used.

**[Table 38]**

| **Effective ingredient** | **Total content (g)** |
|---|---|
| Polyethylene glycol 3350 | 140 |
| Anhydrous sodium sulfate | 9 |
| Potassium chloride | 2.2 |
| Sodium chloride | 5.2 |
| Sodium ascorbate | 48.11 |
| Ascorbic acid | 7.54 |
| **Total amount** | **212.05** |

### Dosing regimen

The effective ingredients were mixed well with each other by content, an appropriate amount of water was added to dissolve them, and then water was added again to be a total amount of 1 L, and mixed well with each other to prepare a preparation solution. For 1 L of total preparation solution, a total of 1 L of water should be taken.

In each Comparative Example and Example, the instructions were to divide the total preparation solution into the corresponding ratio (%) of the previous day's or the day's preparation solution and take it with water, or to divide the effective ingredients in the above Table into the content corresponding to the corresponding ratio (%), separately prepare the previous day's or the day's preparation solution and then take it with water

The dosing regimen (a ratio % of the preparation solution) of each Example is as follows.

**[Table 39]**

| **Comparative Example 18** | **Example 18** |
|---|---|
| Primary: 50% | Primary: 75% |
| On the day before the colonoscopy, 500 mL of the preparation solution was taken and an additional 500 mL of water was drunk. | |
| | On the evening of the day before the colonoscopy, 750 mL of the preparation solution was taken and 750 mL of water was drunk. |
| Secondary: 50% | Secondary: 25% |
| On the morning on the day of the colonoscopy, 500 mL of the preparation solution was taken and an additional 500 mL of water was drunk. | On the morning on the day of the colonoscopy, 250 mL of the preparation solution was taken and 250 mL of water was drunk (Total 500 mL). |

**[Table 40]**

| Classificat ion | | Previous day | | | The day | | | Total | | |
|---|---|---|---|---|---|---|---|---|---|---|
| | | Prepara tion solutio n | Water | Total | Prepar ation soluti on | Water | Total | Prepara tion solutio n | Water | Total amount |
| Comp. Exampl e 18 | % | 50% | 50% | | 50% | 50% | | 100% | 100% | |
| | Volu me | 500 | 500 | 1,000 | 500 | 500 | 1,000 | 1,000 | 1,000 | 2,000 |
| | % | 75% | 75% | | 25% | 25% | | 100% | 100% | |
| Exampl e 18 | Volu me | 750 | 750 | 1,500 | 250 | 250 | 500 | 1,000 | 1,000 | 2,000 |

### Example 19

Compositions were prepared according to the ingredients and contents in the Table below.

**[Table 41]**

| **Ingredients** | **Total content (g)** |
|---|---|
| Ascorbic acid | 40.6 |
| Sodium ascorbate | 9.4 |
| Polyethylene glycol 3350 | 160.0 |
| Potassium chloride | 1.0 |
| Sodium chloride | 2.7 |
| Anhydrous sodium sulfate | 18.0 |
| **Total amount** | **231.7** |

### Dosing regimen

The effective ingredients were mixed well with each other by content, an appropriate amount of water was added to dissolve them, and then water was added again to be a total amount of 1 L, and mixed well with each other to prepare a preparation solution. For 1 L of total preparation solution, a total of 1 L of water should be taken.

In each Comparative Example and Example, the instructions were to divide the total preparation solution into the corresponding ratio (%) of the previous day's or the day's preparation solution and take it with water, or to divide the effective ingredients in the above Table into the content corresponding to the corresponding ratio (%), separately prepare the previous day's or the day's preparation solution and then take it with water

The dosing regimen (a ratio % of the preparation solution) of each Example is as follows.

**[Table 42]**

| **Comparative Example 19** | **Example 19** |
|---|---|
| Primary: 50% | Primary: 80% |
| On the day before the colonoscopy, 500 mL of the | On the evening of the day before the colonoscopy, 800 mL of the |
| preparation solution was taken and an additional 500 mL of water was drunk. | preparation solution was taken and 800 mL of water was drunk. |
| | Secondary: 20% |
| Secondary: 50% | On the morning on the day of the colonoscopy, 200 mL of the preparation solution was taken and 200 mL of water was drunk (Total 400 mL). |
| On the morning on the day of the colonoscopy, 500 mL of the preparation solution was taken and an additional 500 mL of water was drunk. | |

**[Table 43]**

| Classifica tion | | Previous day | | | The day | | | Total | | |
|---|---|---|---|---|---|---|---|---|---|---|
| | | Preparat ion solution | Water | Total | Preparat ion solution | Water | Total | Preparat ion solution | Water | Total amount |
| Comp. Exampl e 19 | % | 50% | 50% | | 50% | 50% | | 100% | 100% | |
| | Vol ume | 500 | 500 | 1,000 | 500 | 500 | 1,000 | 1,000 | 1,000 | 2,000 |
| Exampl e 19 | % | 80% | 80% | | 20% | 20% | | 100% | 100% | |
| | Vol ume | 800 | 800 | 1,600 | 200 | 200 | 400 | 1,000 | 1,000 | 2,000 |

### Example 20

Compositions were prepared according to the ingredients and contents in the Table below.

**[Table 44]**

| **Effective ingredient** | **Total content (g)** |
|---|---|
| Polyethylene glycol 3350 | 150.0 |
| D-sorbitol | 54.6 |
| Sodium picosulfate hydrate | 0.01 |
| **Total amount** | **204.61** |

### Dosing regimen

The effective ingredients were mixed well with each other by content, an appropriate amount of water was added to dissolve them, and then water was added again to be a total amount of 1 L, and mixed well with each other to prepare a preparation solution. For 1 L of total preparation solution, a total of 1 L of water should be taken.

In each Comparative Example and Example, the instructions were to divide the total formulation by the corresponding ratio (%) of the previous day's or the day's formulation and take it with water, or to divide the effective ingredients in the above Table into the content corresponding to the corresponding ratio (%), separately prepare the previous day's or the day's preparation solution and then take it with water. The dosing regimen (a ratio % of the preparation solution) for each Comparative Example and Example is as follows.

**[Table 45]**

| **Comparative Example 20** | **Example 20** |
|---|---|
| Primary: 50% | Primary: 80% |
| On the day before the colonoscopy, 500 mL of the preparation solution was taken and an additional 500 mL of water was drunk. | On the evening of the day before the colonoscopy, 800 mL of the preparation solution was taken and 800 mL of water was drunk. |
| Secondary: 50% | Secondary: 20% |
| On the morning on the day of the colonoscopy, 500 mL of the preparation solution was taken and an additional 500 mL of water was drunk. | On the morning on the day of the colonoscopy, 200 mL of the preparation solution was taken and 200 mL of water was drunk (Total 400 mL). |

**[Table 46]**

| Classificat ion | | Previous day | | | The day | | | Total | | |
|---|---|---|---|---|---|---|---|---|---|---|
| | | Preparat ion solution | Water | Total | Preparat ion solution | Water | Total | Preparat ion solution | Water | Total amount |
| Comp. Example 4 | % | 50% | 50% | | 50% | 50% | | 100% | 100% | |
| | Vol ume | 500 | 500 | 1,000 | 500 | 500 | 1,000 | 1,000 | 1,000 | 2,000 |
| Example 4 | % | 80% | 80% | | 20% | 20% | | 100% | 100% | |
| | Vol ume | 800 | 800 | 1,600 | 200 | 200 | 400 | 1,000 | 1,000 | 2,000 |

### Results of Examples 17 to 20

Randomly selected patients were administered each of the following compositions according to the dosing regimens described in each of the Examples and Comparative Examples, and degree of bowel preparation, endoscopy time, bowel residual amount, ease of dosage, side effects (adverse cases - nausea, vomiting, dizziness), willingness to take the drug again, etc. were evaluated. Patients above were healthy adults who were scheduled for routine outpatient colonoscopy, had no prior adverse reactions to other commercially available bowel preparation agents, and patients having serious medical problems, heart disease, diabetes, or any other potentially serious condition were excluded.

Among the evaluation items, degree of bowel preparation, ease of the examination, residual amount, and examination time (minutes) were evaluated by the clinician who performed the endoscopy.

### Bowel preparation success rate

The degree of bowel preparation was confirmed by colonoscopy. To objectively evaluate the bowel preparation effect, the grade of bowel cleaning effect was evaluated based on the Harefield Cleansing Scale, which categorizes the degree of bowel preparation of each subject into five phases, was evaluated

Specifically, according to the Harefield Cleansing scale, a score of 1 to 3 indicates a successful bowel cleansing, and scores of 4 and 5 indicate an unsuccessful bowel cleansing.

**[Table 47]**

| Grade | Criteria |
|---|---|
| Excellent (1 point) | bowel empty and clean |
| Good (2 point) | presence of clear liquid in the digestive gut |
| Fair (3 point) | presence of brown liquid or small amounts of semisolid residual stool, fully removable by suction or displaceable, thus allowing a complete visualization of the underlying mucosa |
| Poor (4 point) | presence of semisolid stool, only partially removable with a risk of incomplete underlying mucosal visualization |
| Fail (5 point) | presence of semisolid or solid stool, colonoscopy incomplete or had to be stopped |

### Endoscopy time

The time from start to completion of colonoscopy was recorded.

### Residual amount

Bowel water residual amount was measured by summing the residual water in the stomach through the suction channel in the scope during endoscopy.

### Ease of the examination

The ease of endoscopy and polyp identification due to the degree of bowel preparation and reduction of residual fluid was evaluated.

Among the items to be evaluated, ease of dosage, adverse events, and willingness to re-dose were evaluated by questionnaires from the subjects (patients) were evaluated as categorized as follows.
- Evaluation criteria for the ease of dosage: Not difficult (1 point), a little difficult (2 points), a lot difficult (3 points)
- Criteria for evaluating adverse events: Did not occur (1 point), occurred a little (2 points), occurred a lot (3 points)
- Criteria for assessing willingness to re-dose: will take it again (1 point), will not (2 points)

The results are shown in Table 48.

**[Table 48]**

| Classification | | Example 17 | | | Example 18 | | Example 19 | | Example 20 | |
|---|---|---|---|---|---|---|---|---|---|---|
| | | Comp. Exampl e 17-1 | Examp le 17-1 | Exampl e 17-2 | Comp. Examp le 18 | Examp le 18 | Comp. Examp le 19 | Examp le 19 | Comp. Exampl e 20 | Exampl e 20 |
| Total dosage for the day (ml) | | 675 | 450 | 480 | 1000 | 400 | 1000 | 400 | 1000 | 400 |
| Number of the subjects (people) | | 4 | 4 | 3 | 4 | 4 | 4 | 4 | 4 | 4 |
| Bowel preparat ion success rate | Total | 1.75 | 1.50 | 1.33 | 1.75 | 1.50 | 1.75 | 1.50 | 2.50 | 2.25 |
| | Ease of the examinat ion | 2.25 | 1.75 | 1.67 | 2.00 | 1.50 | 2.50 | 2.00 | 2.75 | 2.00 |
| Endoscop ic time (min ute:seco nd) | Caecum arrival | 04:17 | 03:36 | 04:37 | 06:40 | 05:30 | 05:04 | 05:10 | 05:54 | 04:30 |
| | Recovery | 04:32 | 04:04 | 04:32 | 05:30 | 04:16 | 08:31 | 04:05 | 04:25 | 04:05 |
| | Total time | 08:50 | 07:40 | 09:08 | 12:11 | 09:46 | 13: 35 | 09:15 | 10:18 | 08:35 |
| Residual amount (cc) | | 195 | 148 | 160 | 313 | 206 | 238 | 140 | 243 | 148 |
| Ease of dosage | Morning | 2.00 | 1.50 | 1.33 | 1.50 | 1.25 | 2.25 | 1.50 | 1.75 | 1.25 |
| Adverse events | Nausea | 1.50 | 1.75 | 1.33 | 1.50 | 1.50 | 2.25 | 1.50 | 1.25 | 1.00 |
| | Vomiting | 1.00 | 1.00 | 1.33 | 1.25 | 1.25 | 1.75 | 1.25 | 1.00 | 1.00 |
| | Dizzines s | 1.25 | 1.25 | 1.00 | 1.25 | 1.00 | 1.25 | 1.00 | 1.00 | 1.00 |
| | Urgent stool | 1.75 | 1.00 | 1.00 | 1.50 | 1.25 | 1.50 | 1.50 | 1.50 | 1.00 |
| willingness to take the drug again | | 1.00 | 1.00 | 1.00 | 1.25 | 1.25 | 1.25 | 1.00 | 1.25 | 1.00 |

Examples 17 to 20, all of which had a total daily dose of 500 ml or less of a preparation solution and water, were significantly superior to the Comparative Examples in almost every item (i.e., degree of bowel preparation, ease of dosage, willingness to take the drug again, colonoscopy time, residual amount, etc.). In particular, a significant number of colonoscopy patients also undergo gastroscopy, but fasting for at least 8 hours, including water, is recommended during gastroscopy, which is directly contradictory to the method of taking bowel preparation agents for colonoscopy, and thus in terms of improving the subject's safety, residual amount is also an important evaluation criterion. When taking the example composition according to the present disclosure, the residual amount was dramatically improved, and the safety of the subject was very effectively improved.

With the above Examples, the following effects were confirmed.

In all Examples, despite reducing the dosage on the morning on the day of the examination to less than or equal to 700 mL, especially less than or equal to 600 mL, and even less than or equal to 500 mL, not only better degree of bowel preparation but also the degree of mucosal preparation was shown, and since the bowel environment was suitable condition for colonoscopy, the time to reach and retrieve the cecum during endoscopy was shortened, and the time required for the examination was dramatically reduced. In addition, due to the degree of bowel preparation and the reduction of residual fluid, it was easier to secure a view for endoscopy and polyp identification, and the ease of examination for clinicians had also increased.

Furthermore, the ease of performing endoscopy evaluated by the subjects was excellent, the rate of completion of taking the drug increased, and the number of adverse events was reduced, dramatically improving the ease of dosage and confirming a high willingness to take it again.

Considering that most of the compositions used in the Examples are drugs that have already proven their effectiveness and safety through clinical trials, etc. through existing dosing regimens (Comparative Examples) and are already licensed and commercially available in Korea, a new dosing regimen according to the present disclosure has been confirmed that it is more effective and safer than the approved dosage of drugs currently on the market.

A great improvement in safety was also confirmed that adverse events experienced by patients during the course of taking the drug and the amount of water in the bowel are reduced during actual colonoscopy, making it easier to perform colonoscopy, and reducing the risk of colonoscopy by shortening the examination time. In particular, a significant number of colonoscopy patients also undergo gastroscopy, but fasting for at least 8 hours, including water, is recommended during gastroscopy, which is directly contradictory to the method of taking bowel cleansers for colonoscopy, and thus in terms of improving the subject's safety, gastric residual amount is also an important evaluation criterion, and it was confirmed that when taking the example composition according to the present disclosure, safety of the subjects was very effectively improved with a dramatic improvement of gastric residual amount.

## Claims

1. A bowel cleansing composition taken prior to an examination requiring bowel cleansing, wherein
the composition is taken in divided doses the day before and on the day of the examination, and
a total dosage taken on the day of the examination is 700 mL or less.

2. The bowel cleansing composition of claim 1, wherein the total dosage taken on the day of the examination is 500 mL or less.

3. The bowel cleansing composition of claim 1, wherein the total dosage taken the day before and on the day of the examination is 1.0 L to 4.0 L.

4. The bowel cleansing composition of claim 1, wherein the bowel cleansing composition comprises a preparation solution obtained by dissolving a composition in a form of a powder, granule, liquid preparation, tablet, or capsule containing an effective ingredient in a water-soluble solvent; and separately a water-soluble solvent, and
the bowel cleansing composition is administered in a mode by taking the preparation solution, followed by taking the water-soluble solvent separately, wherein the mode is repeated a total of two to four times the day before and on the day of the examination.

5. The bowel cleansing composition of claim 1, wherein the bowel cleansing composition is in the form of any one of a solution, a suspension, and an emulsion.

6. The bowel cleansing composition according of any one of claims 1 to 5, wherein the bowel cleansing composition comprises one or more sugar alcohols selected from xylitol, sorbitol, glycerol, erythritol, threitol, arabitol, ribitol, mannitol, galactitol, fucitol, iditol, inositol, volemitol, isomalt, maltitol, lactitol, maltotriitol, maltotegratol, and polyglycitol as a first effective ingredient;
ascorbic acid or a mixture of ascorbic acid and ascorbate salts as a second effective ingredient; and
a water-soluble solvent,
wherein the content of the first effective ingredient is from 10 g/L to 500 g/L based on the total composition, the content of the second effective ingredient is from 1 g/L to 300 g/L based on the total composition, and the content of the water-soluble solvent is from 1.0 to 3.0 L.

7. The bowel cleansing composition of claim 6, further comprising sodium bicarbonate and/or potassium bicarbonate as a third effective ingredient,
wherein the content of the third effective ingredient is from 0.5 g/L to 5 g/L based on the total composition.

8. The bowel cleansing composition of claim 6, wherein the bowel cleansing composition comprises
110 g of D-sorbitol;
15 g of ascorbic acid;
2.1 g of sodium bicarbonate;
0.9 g of potassium bicarbonate; and
1.0 L to 3.0 L of water,
wherein the water is administered in the form of a solution in which the ingredients are dissolved, or is administered alone.

9. The bowel cleansing composition of any one of claims 1 to 5, wherein the bowel cleansing composition comprises
polyethyleneglycol as the first effective ingredient;
ascorbic acid or a mixture of ascorbic acid and ascorbate salts as the second effective ingredient; and
water-soluble solven,
whereirn the content of the first effective ingredient is from 10 g/L to 500 g/L based on the total composition, the content of the second effective ingredient is from 1 g/L to 300 g/L based on the total composition, and the content of the water-soluble solvent is from 1.0 to 3.0 L.

10. The bowel cleansing composition of claim 9, wherein the bowel cleansing composition comprises
200 g of polyethylene glycol 3350;
9.4 g of ascorbic acid;
11.8 g of sodium ascorbate;
15 g of anhydrous sodium sulfate;
5.382 g of sodium chloride;
2.030 g of potassium chloride; and
1.0 to 3.0 L of water,
wherein the water is administered in the form of a solution in which the ingredients are dissolved, or is administered alone.

11. The bowel cleansing composition of claim 9, wherein the bowel cleansing composition comprises
140 g of polyethylene glycol 3350;
7.54 g of ascorbic acid;
48.11 g of sodium ascorbate;
9 g of anhydrous sodium sulfate;
5.2 g of sodium chloride;
2.2 g of potassium chloride; and
1.0 to 3.0 L of water,
wherein the water is administered in the form of a solution in which the ingredients are dissolved, or is administered alone.

12. The bowel cleansing composition of claim 9, wherein the bowel cleansing composition comprises
160 g of polyethylene glycol 3350;
40.6 g of ascorbic acid;
9.4 g of sodium ascorbate;
18 g of anhydrous sodium sulfate;
2.7 g of sodium chloride;
1 g of potassium chloride; and
1.0 to 3.0 L of water,
wherein the water is administered in the form of a solution in which the ingredients are dissolved, or is administered alone.

13. The bowel cleansing composition according of any one of claims 1 to 5, wherein the bowel cleansing composition comprisesm, based on the total composition,
0.001 g/L to 0.1 g/L of sodium picosulfate or a hydrate thereof;
0.5 g/L to 50 g/L of magnesium oxide;
1 g/L to 50 g/L of citric acid; and
1.0 to 3.0 L of a water-soluble solvent.

14. The bowel cleansing composition of any one of claims 1 to 5, wherein the bowel cleansing composition comprises, based on the total composition,
10 g/L to 500 g/L of polyethylene glycol;
1 g/L to 300 g/L of sorbitol;
0.001 g/L to 1 g/L of sodium picosulfate or a hydrate thereof; and
1.0 to 3.0 L of a water-soluble solvent.

15. The bowel cleansing composition of any one of claims 1 to 5, wherein the bowel cleansing composition comprises, based on the total composition,
5 g/L to 300 g/L of sodium sulfate;
0.1 g/L to 50 g/L of potassium sulfate;
0.1 g/L to 50 g/L of magnesium sulfate; and
1.0 to 3.0 L of a water-soluble solvent.
